# EUROPEAN PATENT SPECIFICATION

(11) **EP 1 605 939 B1**
(45) Date of publication and mention of the grant of the patent: **15.10.2008**
(21) Application number: 04723128.7
(22) Date of filing: 24.03.2004
(51) Int. Cl.: A61K 31/439, A61K 31/52, A61K 31/4015, A61P 35/00

(54) **PHARMACEUTICAL USE OF 1-AZABICYCLO¬ 2.2.2 OCTANES AND A METHOD OF TESTING COMPOUNDS FOR THE ABILITY OF ACTIVATING INACTIVE WT P53**
PHARMAZEUTISCHE VERWENDUNG VON 1-AZABICYCLO¬ 2.2.2 OCTANEN UND VERFAHREN ZUM TESTEN VON VERBINDUNGEN AUF DIE FÄHIGKEIT ZUR AKTIVIERUNG VON INAKTIVEM WT P53
UTILISATION PHARMACEUTIQUE DE 1-AZABICYCLO ¬2.2.2 OCTANES ET PROCEDE D'ESSAI DE COMPOSES CONCERNANT LA CAPACITE D'ACTIVATION DE WT P53 INACTIF

(30) Priority: 24.03.2003 SE 0300812; 24.03.2003 US 456525 P
(43) Date of publication of application: 21.12.2005
(73) Proprietor: APREA AB, S-171 77 Stockholm (SE)
(72) Inventor: STRÖMBLAD, Staffan, S-141 44 Huddinge (SE); BAO, Wenije, S-141 53 Huddinge (SE); SELIVANOVA, Galina, S-169 64 Solna (SE); ISSAEVA, Natalia, S-184 50 Akersberga (SE)
(74) Representative: Allee, Harriet Eva Charlotta
(86) International application number: PCT/SE2004/000452
(87) International publication number: WO 2004/084893

(56) References cited:
- WO-A1-02/24692
- WO-A1-03/070250
- STROMBLAD S., ET AL.: 'Suppresion of p53 Activity and p21WAF1/CIP1 Expression by Vascular Cell Integrin alphaVbeta3 during Angiogenesis' J. CLIN. INVEST. vol. 98, no. 2, July 1996, pages 426 - 433, XP002979236
- STROMBLAD S., ET AL.: 'Loss of p53 Compensates for alpha v-Integrin Function in Retinal Neovascularization' THE JOURNAL OF BIOLOGICAL CHEMISTRY vol. 277, no. 16, 19 April 2002, pages 13371 - 13374, XP002979237
- BYKOV V. J. N., ET AL.: 'Mutant p53-dependant growth suppression distinguishes PRIMA-1 from known anticancer drugs: a statistical analysis of information in the National Cancer Institute database' CARCINOGENESIS vol. 23, no. 12, 2002, pages 2011 - 2018, XP002979238
- BYKOV V. J. N., ET AL.: 'Restoration of the tumor suppressor function to mutant p53 by a low-molecular-weight compound' NATURE MEDICINE vol. 8, no. 3, March 2002, pages 282 - 288, XP002979239

## Description

### Field of the invention

The present invention relates to the use of certain compounds, capable of transferring wild type p53 from an inactive and non-functional conformation into its active conformation, for the preparation of a pharmaceutical composition for use in treating medical conditions wherein wt p53 exists in an inactive state defined herein, and especially malignant melanoma. The invention also relates to a method of testing of compounds for the above-mentioned ability, wherein the level of inactive wt p53 is monitored. The method can be carried out both *in vitro* and *in vivo.*

### Background art

Although the tumour suppressor protein p53 is mutated in most human tumours, wild type (wt) p53 is dominant in malignant melanoma (Hollstein et al., Science, 253: 49-53, (1991); Sparrow et al., Melanoma Res. 1995. 5: 93-100 (1995); and Hartmann et al., Int J cancer, 67: 313-317, (1996)) Petitclerc et al., Cancer Res, 59: 2724-2730 (1999)) found Mab LM609, a function-blocking monoclonal antibody directed to αᵥβ₃ integrin, to block M21 melanoma growth by inducing tumour apoptosis.

Malignant melanoma are typically refractory to apoptosis induction by chemical drugs or radioactivity.

### Summary of invention

The present inventors have found certain compounds to be capable of inducing apoptosis in malignant melanoma cells, which compounds are defined in claim 1 as having a structure according to the formula (I) 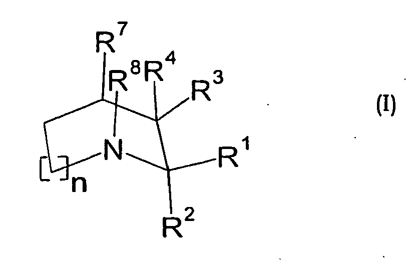 wherein
n is 0,1 or 2;
R¹ and R² are the same or different and are selected from -H, -CH₂-R⁵, -CH₂-O-R⁵, -CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ and -CH₂-O-CO-NHR⁵;
or R¹ and R² are together =CH₂;
R³ and R⁴ are the same or different and are selected from H, -OH, -SH, -NH₂, -NHR⁵ and
-O-CO-C₆H₅;
or R³ and R⁴ together are =O, =S, =NH or =NR⁵;
R⁵ represents the same or different groups selected from H, substituted or non-substituted C1 to C10 alkyl, C2 to C10 alkenyl, C2 to C10 alkynyl, substituted or non-substituted C3 to C12 cycloalkyl, substituted or non-substituted benzyl groups, substituted or non-substituted aryl or mono-, bi-, tricyclic unsubstituted or substituted heteroaromatic ring(s) with one or more heteroatoms and non-aromatic heterocycles wherein
the substituents of the substituted groups are selected from C1 to C10 alkyl, C2 to C10 alkenyl, C2 to C10 alkynyl, halogen, substituted or non-substituted aryl, substituted or non-substituted hetero-aromatic compounds, non-aromatic heterocycles, C1 to C10 alkyloxy, C1 to C10 alkylamino, C2 to C10 alkenylamino, C2 to C10 alkynylamino, COR⁶, CONR⁶ and COOR⁶;
R6 is selected from H, unsubstituted or substituted C1 to C10 alkyl, C2 to C 10 alkenyl or alkynyl, benzyl, aryl, unsubstituted or substituted heteroaromatic rings with one or more hetero-atoms and non-aromatic heterocycles;
R⁷ and R⁸ together form a bridging CH₂-CH₂ moiety; or
R⁷ and R⁸ are both hydrogen; as well as pharmaceutically acceptable salts or prodrugs of the compounds of formula (I).

Where cyclic, the alkyl group is preferably C5 to C10, most preferably C5-C7. Where acyclic, the alkyl group is preferably C1 to C6, more preferably methyl, ethyl, propyl (n-propyl, isopropyl), butyl (branched or unbranched) or pentyl, and most preferably methyl.

As used herein, the term "aryl" means an aromatic group, such as phenyl or naphthyl, or a mono-, bi-, or tricyclic heteroaromatic group containing one or ore heteroatom(s) preferably selected from N, O and S, such as pyridyl, pyrrolyl, quinolinyl, furanyl, thienyl, oxadiazolyl, thiadiazolyl, thiazolyl, oxazolyl, pyrazolyl, triazolyl, tetrazolyl, isoxazolyl, isothiazolyl, imidazolyl, pyrimidinyl, indolyl, pyrazinyl, indazolyl, pyrimidinyl, thiophenetyl, pyranyl, carbazolyl, acridinyl, quinolinyl, benzimidazolyl, benzoimidazolyl, benzthiazolyl, purinyl, azapurinyl, cinnolinyl, pterdinyl.

As used herein, the term "functional groups" means in the case of unprotected: hydroxy-, thiolo-, aminofunction, carboxylic acid and in the case of protected: lower alkoxy, N-, O-, S- acetyl, carboxylic acid ester.

As used herein, the term "heteroaryl" means an aromatic group containing one or more heteroatom(s) preferably selected from N, O and S, such as pyridyl, pyrrolyl, quinolinyl, furanyl, thienyl, oxadiazolyl, thiadiazolyl, thiazolyl, oxazolyl, pyrazolyl, triazolyl, imidazolyl, pyrimidinyl, indolyl, pyrazinyl or indazolyl.

As used herein; the term "non-aromatic heterocycle" means a non-aromatic cyclic group containing one or more heteroatom(s) preferably selected from N, O and S, such as a cyclic amino group such as pyrrolidinyl, piperidyl, piperazinyl, morpholinyl or a cyclic ether such as tetrahydrofuranyl, monosaccharide.

As used herein the term "halogen" means fluorine, chlorine, bromine or iodine, of which chlorine generally is preferred.

As used herein, and unless specified otherwise, the term "substituted" means that the concerned groups are substituted with functional group such as hydroxyl, amine, sulfide, silyl, carboxylic acid, halogen, aryl, etc.

A presently preferred group of compounds are those represented by the formula II 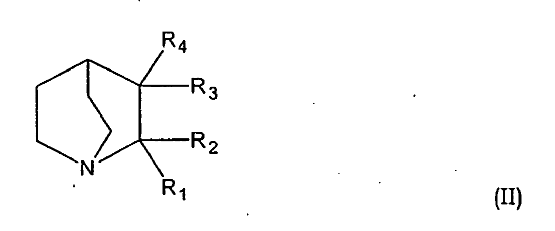 wherein:
R₁ and R₂ are independently selected from hydrogen, hydroxymethyl, or a methylene group linked to the nitrogen atom of an amine-substituted phenyl group, to a nitrogen atom contained in the ring structure of a purine, 8-azapurine, or benzimidazol residue, or R₁ and R₂ may together represent a double bonded methylene group, and;
R₃ and R₄ are independently selected from hydrogen, hydroxyl, and benzoyloxy, or R₃ and R₄ may together represent an oxygen atom being double bonded, with the proviso that when either of R₃ and R₄ is a benzoyloxy group, both R₁ and R₂ are hydrogen.

In the compounds of formula II above, the phenyl group or the nitrogen-containing ring structure of R1, and the benzoyloxy, group of either of R₃ and R₄ can optionally be substituted, such as for example with halogen, methyl, methoxy, amino and/or halomethyl containing 1-3 halogen atoms.

A compound of the invention may be in free form, e.g., amphoteric form, or in salt, e.g., acid addition or anionic salt, form. A compound in free form may be converted into a salt form in an art-known manner and vice versa.

Same compounds are also believed to be capable of inducing apoptosis in angiogenic vascular cells during angiogenesis, thereby inducing regression of angiogenic blood vessels and blocking various disease states dependent on blood vessel formation and/or vascular cell survival, including the growth and metastasis of various human tumours; adult blindness, e.g. caused by diabetic retinopathy or macular degeneration; psoriasis; states of chronic inflammation, e.g. rheumatoid arthritis; hemangioma; and obesity.

In one aspect the present invention provides a method of treating malignant melanoma and/or inhibiting undesired angiogenesis, comprising administrating to a mammal in need thereof a pharmaceutically efficient amount of a compound selected from compounds having a structure according to the formula I.

The present inventors have also found a previously unknown and most unexpected inactive conformation state of wt p53 protein in tumour cells. The inactive wt p53 conformation has now been found in malignant melanoma cells. More importantly, said inactive wt p53 conformation can be reverted into a functional active conformation, capable of inducing apoptosis. Based on these findings, the present invention, in another aspect, provides a method of testing substances for their ability in transforming inactive wt p53 conformation into an active wt p53 conformation, and thus for finding suitable substances for treating malignant melanoma. The method is defined in claim 6. Since same inactive wt p53 conformation also is believed to be present in angiogenic vascular cells during angiogenesis, same substances are also thought to be useful in inhibiting angiogenesis in pathological conditions known to involve angiogenesis.

### Brief description of the attached drawings

FIGURE 1A shows the percentage of apoptotic cells as function of time in human melanoma cells M21 expressing integrin αᵥβ₃ and M21L lacking integrin αᵥβ_{3.}
FIGURE 1B shows the percentage of apoptotic cells as function of time in human melanoma cells M0, lacking integrin αᵥβ₃; M0-2bαV, M0 cells overexpressing a chimer of the extracellular domain of the integrin IIb subunit with the cytoplasmic tail of the integrin αV subunit; and M0-αV cells expressing high levels of the integrin αV subunit.
FIGURE 1C illustrates the cell surface expression of integrin αᵥβ₃ and the integrin β₃ subunit in different human melanoma cell lines.
FIGURE 1D shows the results of an analysis for cleavage of caspase-8 and caspase-9.
FIGURE 2A; upper panel depicts the specific p53 DNA binding activity in M21 and M21L cells collected from 3D-collagen using an electrophoretic gel shift assay (EMSA) with a p53 supershift using Pab 421; Lower panel presents the protein levels of p53 as compared to actin as a loading control.
FIGURE 2B, same as figure 2A upper panel, but with M0 and M0αv cells.
FIGURE 2C shows acetylation of p53 at lysine 382 and the protein levels of PUMA, Apaf1, Bax and Bcl-in M21 (av+) and M21L (αv-) cells after culture in 3D-collagen for up to 7 d in 3D collagen were detected by Western blotting.
Data shown in FIGURE 2D represents Pab 1620 and Pab 240 reactivity as percentage of D01 reactivity (total p53) in M21 (αᵥβ₃+) cells and M21L (αᵥβ₃-) cells.
FIGURE 3A upper panel shows the percentage of apoptotic cells in M21 cells (αᵥβ₃+), M21L cells (αᵥβ₃-) and a number of clones of M21L cells (αᵥβ₃-) stably transfected with a dominant negative p53-His175, including M21Lp53His175-0, M21Lp53His175-1, M21Lp53His175-8 and M21Lp53His175-32. Lower panel shows p53 DNA binding activity in these clones after 5 d in 3D collagen.
FIGURE 3B illustrates tumour growth *in vivo* of cells used in Figure 3A.
FIGURE 3C the graph displays values of mean tumour volumes over time out of 5-7 animals for each tumour cell type shown in the Figure.
FIGURE 3D depicts a bar graph showing the tumour wet weights after 25 d of tumour growth in mice of M21, M21L, M21Lp53His175-0 and M21Lp53His175-8.
FIGURE 3E M21 (αv+), M21L (αv-) and six individual M21L p53siRNA clones (Lp53siRNA) melanoma cells were cultured in 3D-collagen for the indicated times. Apoptosis was detected by Annexin-V staining. The blots for p53 and actin protein levels are shown in the vertical display for the cells indicated immediately to the left of each lane. The displayed results are representative among three independent experiments.
FIGURE 4A shows the results of examination of p53 conformation with Pab 1620 and Pab240 in M21 and M21L melanoma cells incubated with or without 100 µM of PRIMA-1 for 5d.
Figure 4B upper panel shows the results of examination of p53 conformation with Pab 1620 and Pab 240 in AA melanoma cells under 2D culture conditions (d 0) and after 5 d within 3D collagen. Lower panel displays examination of the p53 conformation after 5 d in 3D collagen with or without 100 µM PRIMA1.
Figure 4C upper panel shows quantification of apoptosis by Annexin V staining in M21 melanoma cells after 16, 24, 36, and 48 h in 3D collagen with or without treatment with 100 µM PRIMA-1. Lower panel shows the results of the same treatment under two-dimensional culture conditions.
FIGURE 4D shows M21 human melanoma tumours grown s.c. in nude mice treated with a single injection of PRIMA-1, lysed and analyzed for acetylation of p53 at lysine 382, phosphorylation of p53 at Ser 15 and the protein levels of PUMA, Apaf1 and by Western blotting 24 and 36 h after PRIMA-1 injection.
FIGURE 4E shows that caspase 9, but not Caspase 8, is activated by PRIMA-1 in M21 melanoma tumors treated as described in Figure 4D.
FIGURE 4F shows that PRIMA-1 induced apoptosis is blocked by the caspase 9 inhibitor Z-LEHD-FMK x TFA (C1355) in 3D-collagen
FIGURE 4G upper panel shows mean tumour volumes over time of M21 human melanoma cells grown in C57/BL nude mice treated with or without PRIMA-1 (100 mg/kg) for 6 d. Lower panel shows mean tumour volumes over time of C8161 human melanoma cells grown in the C57/BL nude mice treated with or without PRIMA-1 (100 mg/kg) for 6 d.
In FIGURE 4H, the tumour wet weights after 25 d of M21 melanoma tumour formation with or without PRIMA-1 treatment as described in Fig. 4G are displayed.

### Detailed description of the invention

p53 is mutated in most human tumours, but the rare p53-mutation frequency in malignant melanoma is puzzling. However, malignant melanoma express high levels of integrin αᵥβ₃, an integrin that promotes melanoma cell survival (Montgomery et al., PNAS 91: 8856-8860 (1994); Petitclerc et al; 1999; Hsu et al., Am J Pathol. 153: 1435-1442 (1998)) and suppresses p53-activity in endothelial cells (Strömblad et al., J. Clin. Invest. 98:426-433 (1996); Strömblad et al., J. Biol. Chem. 277: 13371-13374 (2002)). The present inventors now suggest that integrin αᵥβ₃ suppresses melanoma wt p53-activity via induction of a previously unknown inactive, unfolded wt p53-conformation. In tumor cells, said integrin αᵥβ₃-regulated p53-activity has been found to govern melanoma cell survival and tumour growth. Dominant negative p53 (His 175) as well as p53siRNA was found to restore cell survival and tumor growth of melanoma cells lacking integrin αᵥβ₃, suggesting that the integrin-mediated inactivation of p53 controls melanoma cell survival. The compound 2,2-bis(hydroxymethyl)-1-azabicyclo[2.2.2]octan-3-one, also referred to as PRIMA-1, previously known from W00224692 to have the ability to revert mutant p53-conformation to an active state, surprisingly restored the active conformation of wt p53 in melanoma cells, induced apoptosis and blocked melanoma tumor growth in a wt p53-dependent manner. These results may explain the lack of need for p53-mutations in malignant melanoma, and points to a novel principle for melanoma therapy.

Strömblad, S. et al. in J. Clin. Invest. 98:426-433 (1996**),** cited above, reported that proliferative endothelial cells become apoptotic in response to antagonists of integrin αᵥβ₃, leading to the regression of angiogenic blood vessels, and thereby blocking the growth of various human tumours, and that the blocking of integrin αᵥβ₃ lead to activation of p53.

Based on the findings reported in the above paper, and the findings disclosed in the present application, the inventors also believe the present compounds to be capable of inducing apoptosis in proliferative endothelial cells during angiogenesis, thereby inducing regression of angiogenic blood vessels and blocking various disease states dependent on blood vessel formation and/or vascular cell survival, including the growth and metastasis of various human tumours; adult blindness, e.g. caused by diabetic retinopathy or macular degeneration; psoriasis; states of chronic inflammation, e.g. rheumatoid arthritis; hemangioma; and obesity. Consequently, the same inactive wt p53 conformation as disclosed herein is also believed to be present in such proliferative endothelial cells during angiogenesis.

While the inactive conformation of wt p53 in melanoma cells is believed to be induced by the integrin αᵥβ₃, the mechanism underlying the induction of any similar inactive wt p53 states in other tissues could be different. However, since the actual mechanism of action of the present compounds is not fully understood, it is also conceivable that the same compounds could be useful for treating other pathological conditions wherein inactive wt p53 is present.

Substances useful in treating other pathological conditions wherein inactive wt p53 is present can be found by means of the generic method of the present invention.

As used herein, the term "inactive wt p53 conformation" is used interchangeably with the term "inactive, unfolded wt p53 conformation" to designate a conformation of wt p53 which is reactive with the monoclonal antibody Pab 240, but does not react with the monoclonal antibody Pab 1620. Furthermore, the inactive conformation also has the ability of being transformed into an active wt p53 conformation by means of the above chemical compound designated PRIMA-1. Inactive wt p53 conformation is further characterized as being incapable of binding to DNA, and incapable of mediating apoptosis.

Pab 240 has previously only been known to be reactive with mutant p53 under non-denaturing conditions, while being equally reactive with mutant p53 and wt p53 under denaturing conditions. However, the present inventors have now found Pab 240 also to be reactive to the inactive state of wt p53, defined herein, under non-denaturing conditions.

Integrin αᵥβ₃ plays a critical role for cutaneous melanoma progression displaying increased expression in the vertically growth phase melanoma lesions compared to more benign radial growth phase lesions (Albeda et al., Cancel Res. 50: 6757-6764 (1990)). In experimental models, expression of integrin αᵥβ₃ promotes melanoma cell survival, tumour growth and conversion from radial to the vertical growth phase while blocking of integrin αᵥβ₃ inhibits melanoma tumour growth by inducing apoptosis (Felding-Habermann, J Clin Invest. 89: 2018-22 (1992); Petitclerc et al., 1999; Hsu et al., 1998), implicating that integrin αᵥβ₃ functionally regulates melanoma cell survival. Indeed, integrin αᵥβ₃ has also been found to promote melanoma cell survival in a 3-dimensional environment in *vitro* (Montgomery et al., 1994). But, it is still unclear how integrin αᵥβ₃ can control melanoma cell survival.

The tumour suppressor protein p53 functionally induces apoptotic cell death in response to various types of stresses (Oren, Cancer Biol. 5: 221-227 (1994); Ko and Prives, Genes Dev. 10: 1054-1072 (1996); Levine, Cell 88: 323-331 (1997)). Activated p53 can trigger apoptosis via two pathways (Vousden, Cell 103: 691-694 (2000)). One of them is associated with death receptors and activation of caspase-8 (Ashkenazi and Dixit, Science 281: 1305-8 (1998)), while the other apoptosis pathway is mediated through mitochondrial cytochrome c release and subsequent activation of Apaf1 and caspase-9 (Green and Reed, Science 281: 1309-12 (1998)). Furthermore, p53 acts as a specific DNA binding protein to activate or repress expression of a variety of genes, including transcriptionally activating bax (Miyashita et al., Cell 80: 293 (1995)), *PIG3* (p53-inducible gene 3, Venot et al., EMBO J. 17: 4668-79 (1998)), *PUMA* (p53 upregulated modulator of apoptosis, Nakano and Vousden, Mol Cell. 7: 683-694 (2001)), and Apaf-1(apoptotic protease-activating factor 1; (Vousden and Lu, 2002)) and transcriptionally repressing bcl-2 (Zhan et al., Oncogene 9: 3743-51 (1994)). These downstream targets are functionally involved in certain p53-mediated apoptotic processes.

A new mechanism for p53-induced apoptosis acting directly on the mitochondrial membrane has recently been reported by Mihara, A. et al. in "p53 has a direct apoptogenic role at the mitochondria", Molecular Cell, published 27 January 2003. In accordance therewith, p53, by moving rapidly to the mitochondria, effectively 'jump starts' and amplifies its slower-starting transcription-dependent effect on apoptosis.

In addition, an antagonist of integrin αᵥβ₃ induces proliferative endothelial cell apoptosis, which is associated with activation of p53 (Strömblad et al., 1996; Strömblad et al., 2002). Although p53 is mutated in most human tumors, wt p53 is dominant in malignant melanoma (Hollstein et al., 1991; Sparrow et al., 1995; Hartmann et al., 1996). In addition, malignant melanoma are typically refractory to apoptosis induction by chemical drugs or radioactivity.

### Experimental

In order to demonstrate that integrin αᵥβ₃-mediated regulation of p53 regulates melanoma cell survival, the following experimentation, which will be described in more detail hereinafter, was performed. First, M21 human melanoma cells expressing integrin αᵥβ₃, and the M21L subpopulation lacking integrin αᵥβ₃, were analyzed in a 3-dimensional collagen model mimicking the pathophysiological dermal environment. It was found that integrin αᵥβ₃ inhibited p53 activity by inducing an inactive wt p53 conformation. Furthermore, overexpression of a dominant negative p53 mutant (His 175) or p53 siRNA restored M21L (αᵥβ₃-) cell survival and tumour growth, suggesting that p53 is a key target for integrin αᵥβ₃ promoting melanoma cell survival. Importantly, PRIMA-1 could restore the active conformation of wt p53 in M21 cells and thereby induce melanoma cell apoptosis and suppress melanoma tumour growth. These results reveal a novel principle for treatment of melanoma by reactivation of wt p53.

### Integrin αᵥβ₃ promotes melanoma cell survival

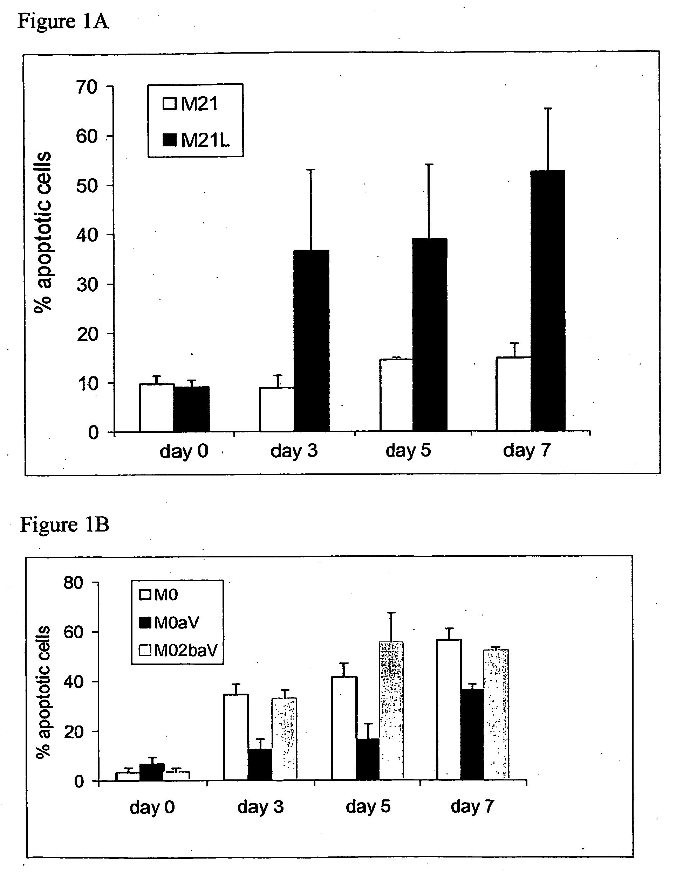

With reference to Figure 1A, the maternal human melanoma cells M21, expressing integrin αᵥβ₃, and a subpopulation, M21L, lacking integrin αᵥβ₃, were cultured in a three-dimensional collagen gel (3D-collagen). At indicated times, apoptosis was detected by FITC-Annexin-V staining. FACS analysis showed that M21L cells lacking αᵥβ₃ became apoptotic to a much higher degree than M21 cells (αᵥβ₃+), suggesting that integrin αᵥβ₃ expression is critical for melanoma cell survival.

The maternal human melanoma cells M0, lacking integrin αᵥβ₃; M0-2bαV, M0 cells overexpressing a chimer of the extracellular domain of the integrin IIb subunit with the cytoplasmic tail of the integrin αV subunit; and M0-αV cells expressing high levels of the integrin αV subunit, were cultured in a 3D-collagen. At indicated times, apoptosis was examined by FITC-Annexin-V staining. As shown in Figure 1B, FACS analysis showed that M0αV cells (expressing αᵥβ₃) survived well while both M0 and M02bαV cells underwent apoptosis to a higher degree at all time points. This indicates that the integrin αV-subunit extracellular ligand binding part is important for melanoma cell survival.

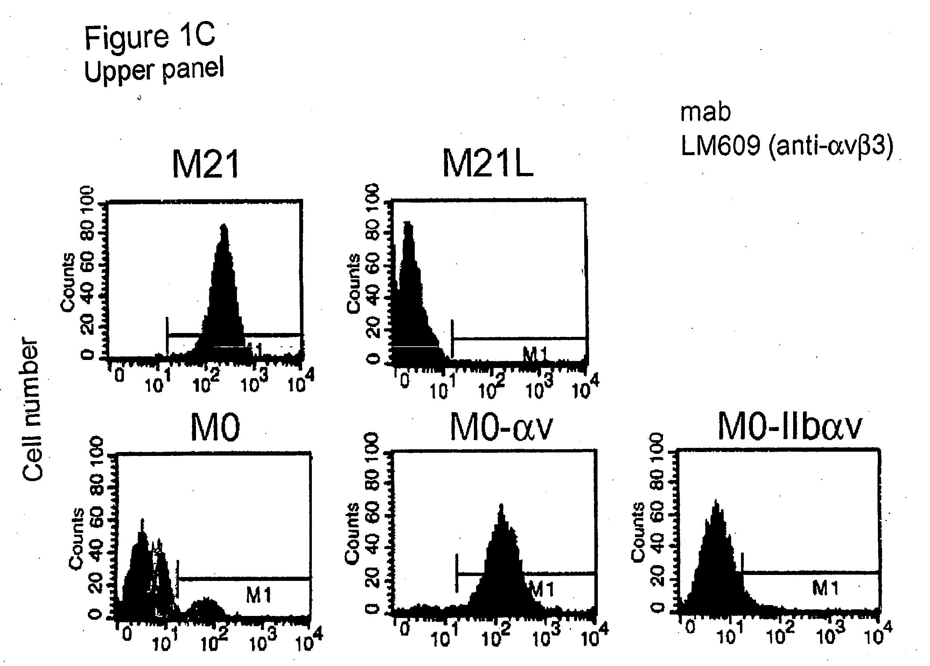

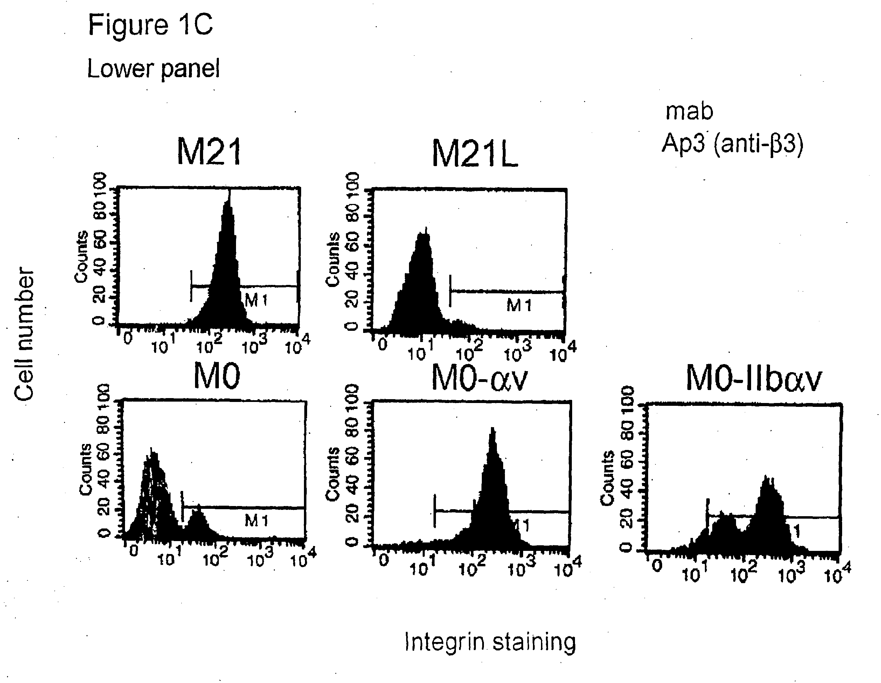

Cell surface expression of integrin αᵥβ₃ and the integrin β₃ subunit was analyzed in the human melanoma cell lines M21, M21L, M0, M0-αV and M0-2bαV stained with anti-αᵥβ₃ Mab LM609 and anti-β₃ Mab AP3, respectively. As can be seen from Figure 1C, FACS analysis showed that both αᵥβ₃ and the β₃ subunit were expressed in all M21 and M0αV cells but rarely expressed in M21L and M0 cells. While integrin β₃ was expressed in virtually all M0-2bαV cells, integrin αᵥβ₃ was not present.

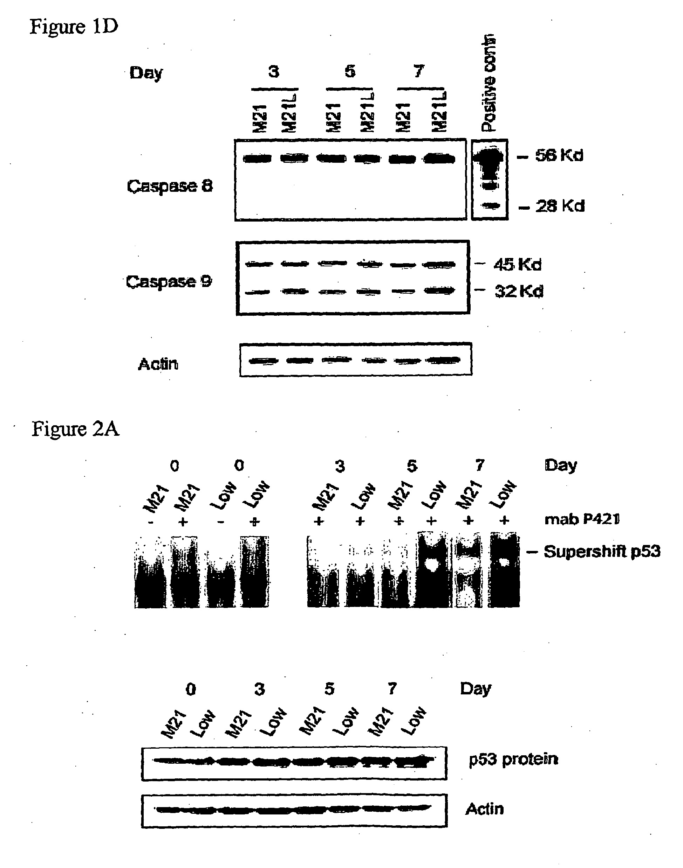

It has been suggested that unligated integrin αᵥβ₃ might cause apoptosis by direct induction of caspase-8 cleavage (Stupack et al, J. Cell Biol. 155: 459-470 (2001)). Therefore, lysates prepared from M21 and M21L cells out of 3D-collagen after 3, 5 and 7 days were analysed by Western blotting for cleavage of caspase-8 and caspase-9. With reference to Figure 1D, no cleavage of caspase-8 in these melanoma cells was observed, while a control with cycloheximide-treated Jurkate cells displayed caspase-8 cleavage. However, caspase-9 was cleaved to a higher degree in M21L cells (αᵥβ₃-) as compared to M21 (αᵥβ₃+) cells, indicating that lack of integrin αᵥβ₃ caused apoptosis in M21L cells and that this apoptosis might be mediated by a mitochondrial apoptotic pathway involving caspase-9 but did not involve any detectable activation of caspase-8.

### Integrin αᵥβ₃ induces an unfolded conformation of wt p53 and suppresses wt p53 activity

The specific p53 DNA binding activity was examined in M21 and M21L cells collected from 3D-collagen using an electrophoretic gel shift assay (EMSA) with a p53 supershift using Pab 421. The p53 activity was similar in M21 and M21L cells at day 0. However, as shown in upper panel of Figure 2A, M21L cells lacking integrin αᵥβ₃ displayed an increased p53 activity after culturing in 3D-collagen while M21 cells (αᵥβ₃ +) did not.

The protein levels of p53 were analyzed by Western blotting using anti-p53 Pab. With reference to lower panel of Figure 2A, the p53 protein levels in M21 and M21L cells showed no difference within 7 d in 3D-collagen (examination performed on day 0, 3, 5 and 7). The levels of actin were examined as a loading control. This indicates that integrin αᵥβ₃ regulates p53 activity without influencing p53 protein levels.

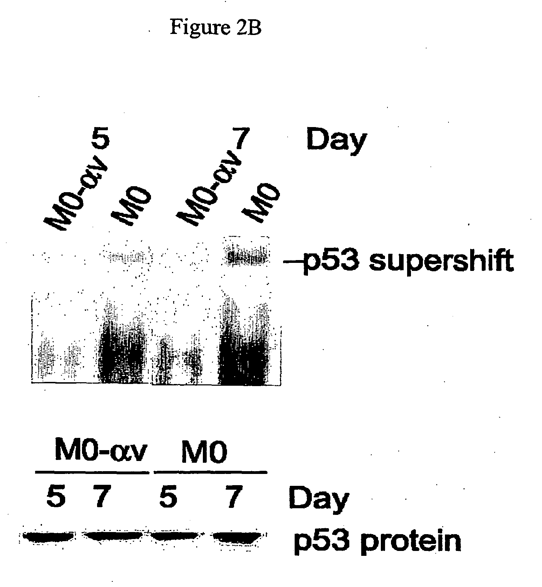

The specific p53 DNA binding activity was examined in M0 and M0αV cells after incubation within 3D-collagen for 5 and 7 days, respectively. The EMSA assay showed that the p53 activity was higher in M0 cells lacking integrin αᵥβ₃ than that in M0αV cells (αᵥβ₃+) (see Figure 2B). This verifies the role for integrin αᵥβ₃ regulating melanoma cell p53 activation state in a second cell system.

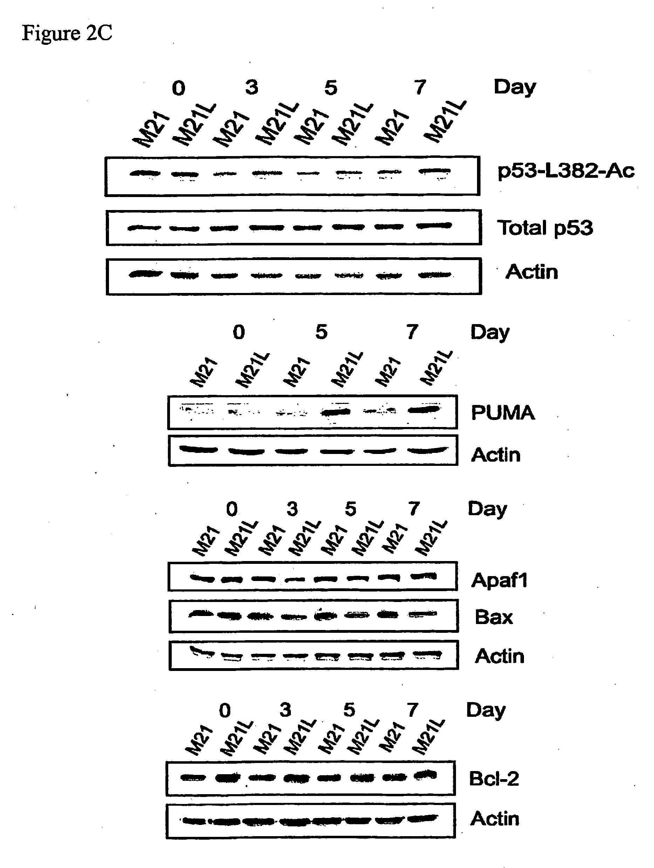

Acetylation of p53 at lysine 382, a known acetylation site for p53 activation was examined by Western blotting using anti-acetylation p53-L382 pab. As can be seen from Fig. 2C, the levels of acetylated p53 were reduced in M21 cells (αᵥβ₃+) as compared to M21L cells (αᵥβ₃-), while total p53 levels remained the same. Furthermore, PUMA was upregulated in M21L (αᵥβ₃-) cells in parallell with p53 activation, but not in M21 (αᵥβ₃+) cells, indicating that the p53 activity might transcriptionally activate PUMA.

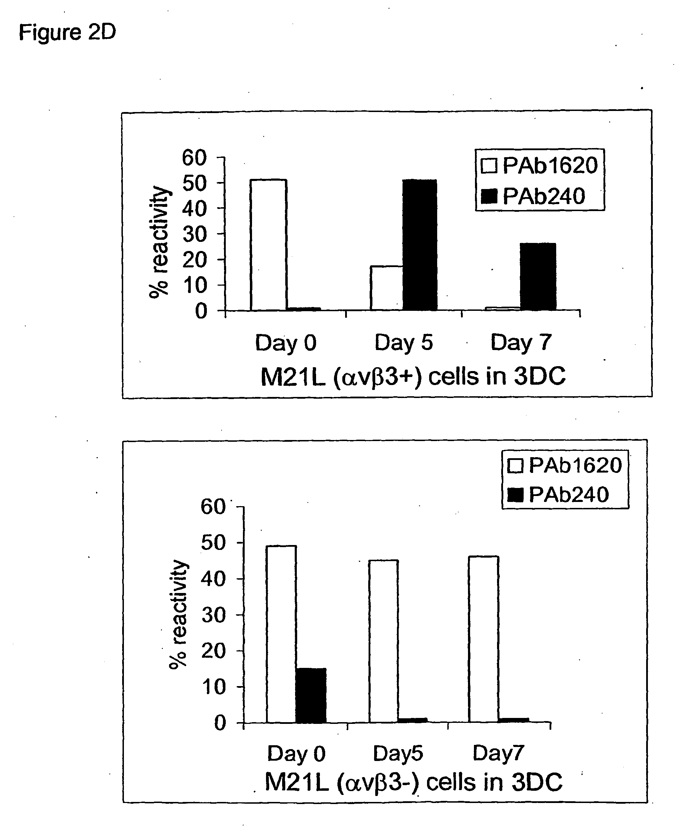

With reference to Figure 2D, the conformation of p53 was analyzed by ELISA using Pab1620 recognizing the active, folded p53 conformation; Pab 240 recognizing an inactive, unfolded p53 conformation and; DO1 recognizing both folded and unfolded p53 conformations. Data shown in Figure 2D represents Pab 1620 and Pab 240 reactivity as percentage of DO1 reactivity (total p53). It was found that both M21 cells (αᵥβ₃+) and M21L (αᵥβ₃-) displayed an active p53 conformation in regular two-dimensional culture (day 0). However, p53 switched to an inactive unfolded p53 conformation after incubation in 3D-collagen-incubated M21 cells (αᵥβ₃+), while M21L (αᵥβ₃-) maintained an active p53 conformation. This suggests that integrin αᵥβ₃ regulates wt p53 conformation in melanoma cells in a pathophysiological 3-dimensional environment and that the lack of p53-activity in cells expressing integrin αᵥβ₃ is caused by an unfolded, inactive p53-conformation.

### Integrin αᵥβ₃-regulated p53 controls melanoma cell survival and melanoma tumor growth

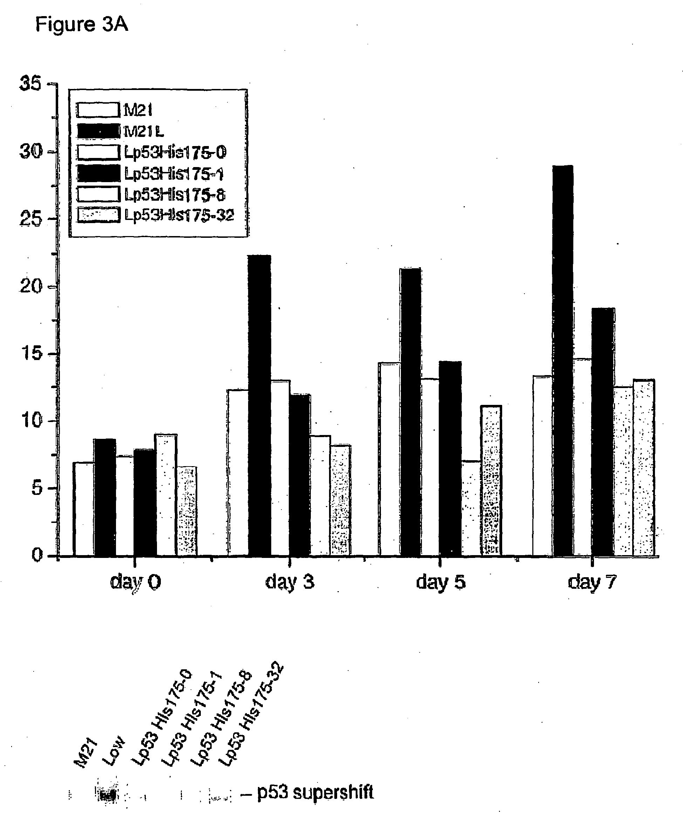

M21L cells (αᵥβ₃-) were stably transfected with a dominant negative p53-His175 and a number of clones, including M21Lp53His175-0, M21Lp53His175-1, M21Lp53His175-8 and M21Lp53His175-32 were identified by examining p53 activity using EMSA after incubation in 3D-collagen for 5 d. The p53 activity in these clones was reduced to a similar level as in M21 cells expressing αᵥβ₃ (lower panel in Figure 3A). The M21L-p53His175 clones, M21L and M21 cells were incubated within 3D-collagen and apoptosis was examined by FITC-Annexin-V staining at times indicated in Figure 3A. FACS analysis showed that the number of apoptotic cells was lower in the M21L carrying dn p53 clones as compared to M21L, and that the rate of apoptosis in the M21L-dnp53 clones was similar to that in M21 cells expressing integrin αᵥβ₃, indicating that the over-expression of dominant negative p53 could rescue M21L cells from apoptosis to a similar degree as expression of integrin αᵥβ₃ itself.

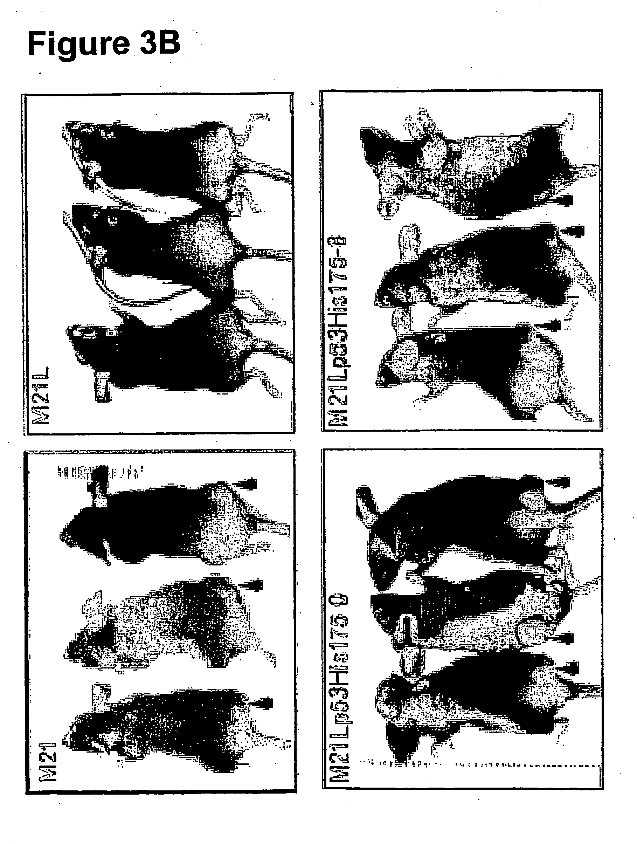

Role of integrin-regulated p53 in melanoma tumour growth was investigated *in vivo*. Results are shown in Figure 3B. Melanoma cells (1 x 10⁶), including M21L, M21 and M21L-dn p53 clones M21Lp53His175-0 and M21Lp53His175-8, were injected s.c. into the back of 6-week-old C57/BL nude mice. Each type of injected cell type included 7 mice. The tumours were allowed to grow for 25 d. Tumours were found to be formed by M21Lp53His175-0, M21Lp53His175-8, and M21 cells (αᵥβ₃+), while M21L cells lacking integrin αᵥβ₃ typically did not form tumours within this time.

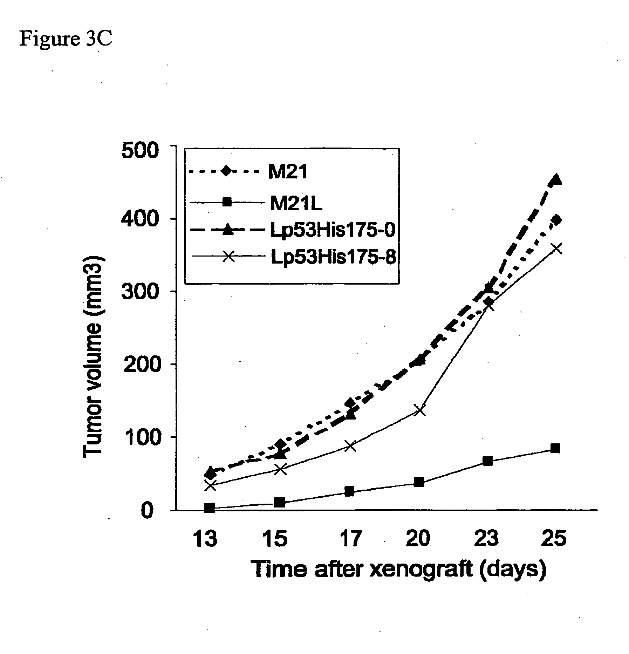

Tumour volumes were monitored for melanoma growth, as described in the preceding paragraph. Tumour volume over time was determined by the formula: width² × length × 0,52. With reference to Figure 3C, the displayed values are mean tumour volumes out of 5-7 animals for each tumour cell type. In the Figure, ◆ represents M21; □ M21L; ▲ Lp53His175-0; and * Lp53His175-8. M21Lp53His175-0 and M21Lp53His175-8 formed large tumours similar in size to M21 tumours. However, M21L cells lacking integrin αᵥβ₃ typically grew only minimal or developed no tumours.

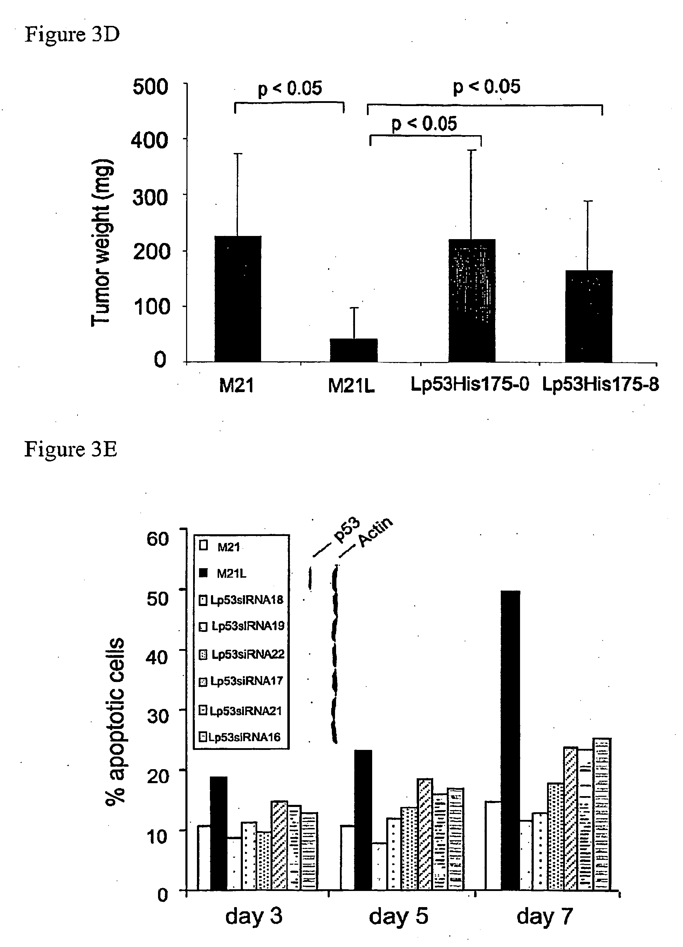

Bar graph showing the mean values plus minus S.D. tumour wet weights of M21, M21L, M21Lp53His175-0 and M21Lp53His175-8 are contained in Figure 3D. Cells were grown s.c. in nude mice for 25 d as described in Fig 3B.

M21L cells (αᵥβ₃-) were stably transfected with p53-siRNA and a number of clones, including M21Lp53siRNA16, M21Lp53siRNA17, M21Lp53siRNA18, M21Lp53siRNA19, M21Lp53siRNA21, and M21Lp53siRNA22, were identified by examining p53 protein levels using Western blot. (vertical panel in Figure 3E). The M21Lp53siRNA clones, M21L and M21 cells were incubated within 3D-collagen and apoptosis was examined by FITC-Annexin-V staining at times indicated in Figure 3E. FACS analysis showed that the number of apoptotic cells was lower in the M21L carrying dn p53 clones as compared to M21L, and that the rate of apoptosis in the M21L-p53siRNA clones was similar to that in M21 cells expressing integrin αᵥβ₃, indicating that the over-expression of p53siRNA could rescue M21L cells from apoptosis to a similar degree as expression of integrin αᵥβ₃ itself.

### Example

In this example it will be demonstrated that PRIMA-1 induces an active p53 conformation in integrin αᵥβ₃ positive melanoma cells, induces p53 transcriptional targets, promotes melanoma cell apoptosis, including specific Caspace activation, and suppresses melanoma tumour growth in *vivo.*

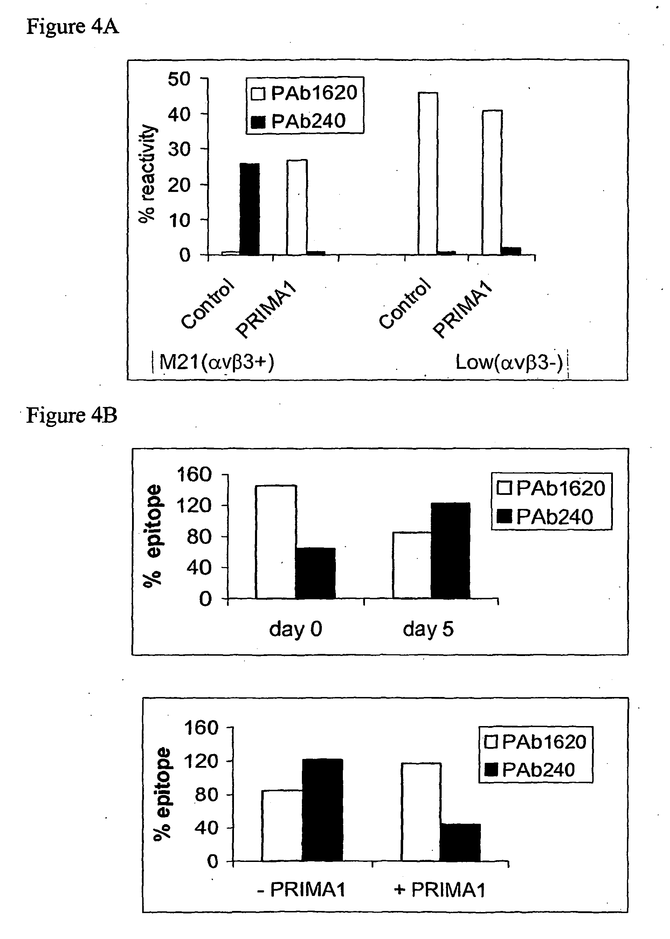

PRIMA-1 is a substance known from WO0224692 to be able to reactivate the apoptosis-inducing function of mutant p53 proteins. To test if PRIMA-1 could affect also the unfolded, inactive conformation of wt p53 in melanoma cells enforced by integrin αvβ3, M21 cells (avβ3+) and M21L cells (αᵥβ₃-) were incubated with or without 100 µM PRIMA-1 in 3D-collagen for 7 d. The p53 conformation was examined as described above. The results are presented in Figure 4A. As can be seen, PRIMA-1 could restore active p53 conformation from an unfolded inactive state in M21 cells (αᵥβ₃+), while the p53 conformation in control M21L cells (αᵥβ₃-) was not influenced by PRIMA-1. This demonstrates in an independent way that wt p53 in melanoma cells can undergo conformational changes between unfolded and folded states. More importantly, these results surprisingly indicates that wt p53 unfolded conformation in melanoma cells can be reverted to active conformation by PRIMA-1. To this end, p53 conformation was also analyzed in AA human melanoma cells expressing αvβ3 integrin and wt p53 before and after incubation within 3D collagen (Figure 4B). The results in the upper panel show that p53 in AA cells had an active conformation under 2D culture conditions (day 0) and that incubation within 3D collagen induced an inactive conformation. However, importantly, lower panel shows that treatment of AA melanoma cells with PRIMA-1 enforced p53 into an active conformation within 3D collagen. This shows that PRIMA-1 can be used to convert wt p53 from inactive to an active conformation in different melanoma cells.

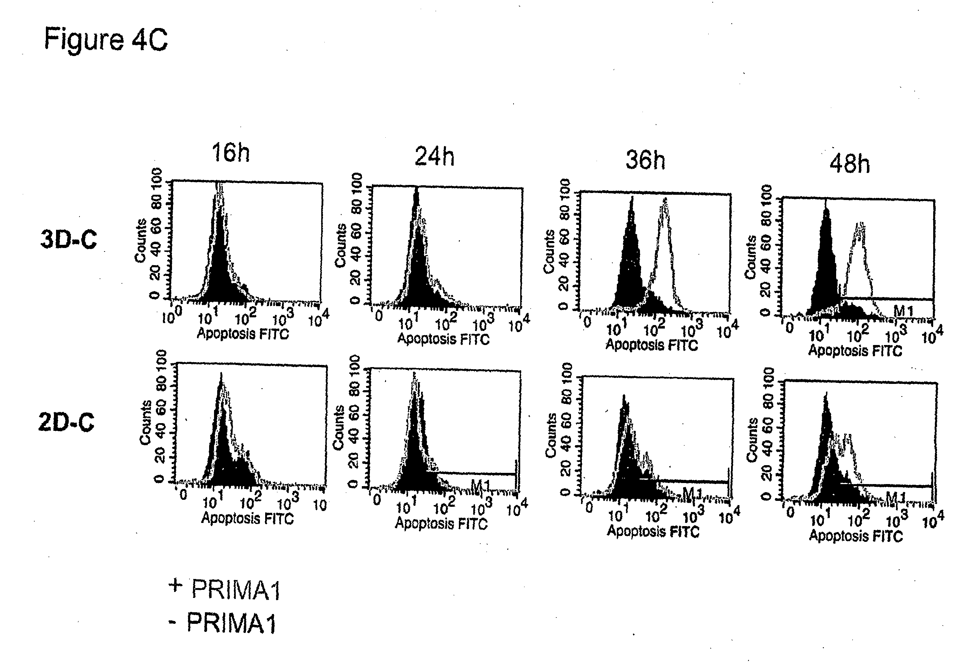

Apoptosis was analyzed in M21 cells grown under 2D culture conditions or within 3D-collagen with or without PRIMA-1 treatment for 16, 24, 36 and 48 h. Results are shown in Figure 4C. PRIMA-1 promoted apoptosis in M21 cells (αᵥβ₃+) within 3D collagen to a much higher degree than in 2D culture, suggesting that regulation of the p53 activation state controls melanoma cell survival selectively within 3D environments. This also verifies the results using dn p53 and p53-siRNA (Fig 3) in an independent manner.

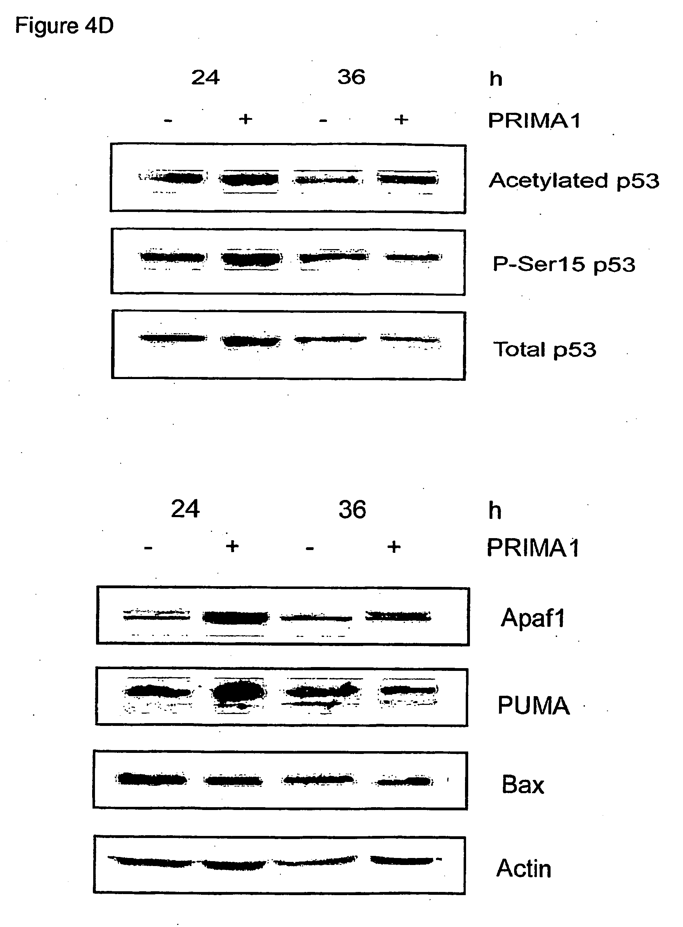

Figure 4D shows acetylation of p53-Lys382, phosphorylation of p53-Ser15, and protein levels of Apaf-1, PUMA and Bax in M21 human melanoma cell tumors grown in nude mice 24 h and 36 h after a single injection of PRIMA-1, all detected by Western blot. The results show that PRIMA-1 induces p53 actetylation as well as Apaf-1 and PUMA protein levels in melanoma tumors in vivo. Thus, acetylation of p53-Lys382 is potentially involved in p53 activation by PRIMA-1, while Apaf-1 and PUMA are potential downstream mediators of PRIMA-1- and p53-induced melanoma cell apoptosis in vivo.

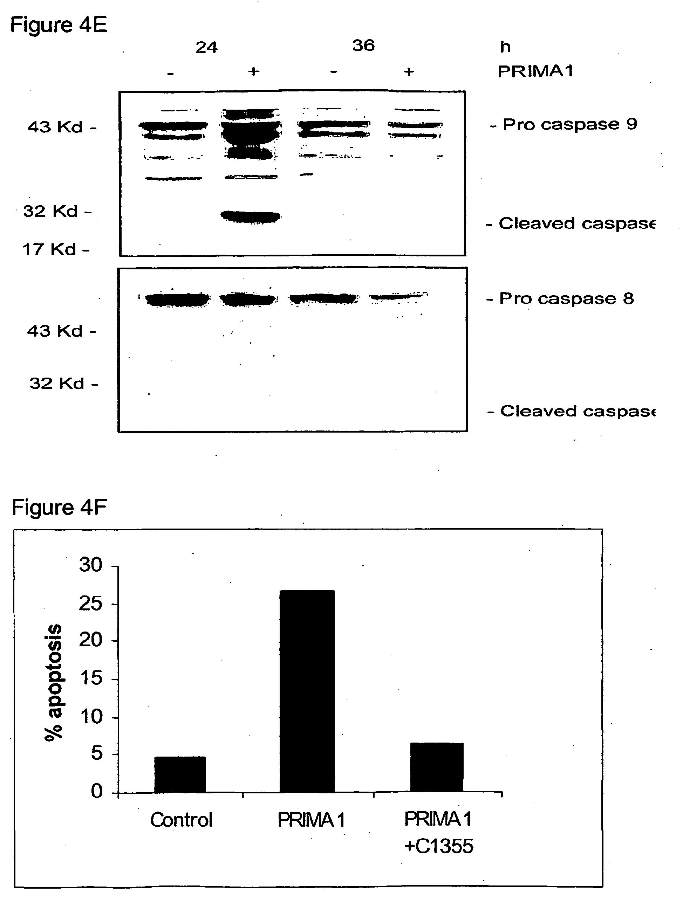

Figure 4E shows activation of Caspase 9, but not Caspase 8, in M21 human melanoma cell tumors grown in nude mice 24 h and 36 h after a single injection of PRIMA-1, detected by Western blot. The reults show that PRIMA-1 induces. Caspase 9 is therefore a potential downstream effector of PRIMA-1- and p53-induced apoptosis inmelanoma cells in vivo.

Figure 4F shows detection of apoptosis by Annexin-V staining of M21 emlanoma cells after 36 h within 3D collagen with or without 80 µM of PRIMA-1 with or withour the Caspase-9 inhibitor Z-LEHD-FMK x TFA (C1355). The results show that PRIMA-1 induces melanoma cell apoptosis in 3D collagen may be dependent on active Caspase 9 as a downstream mediator.

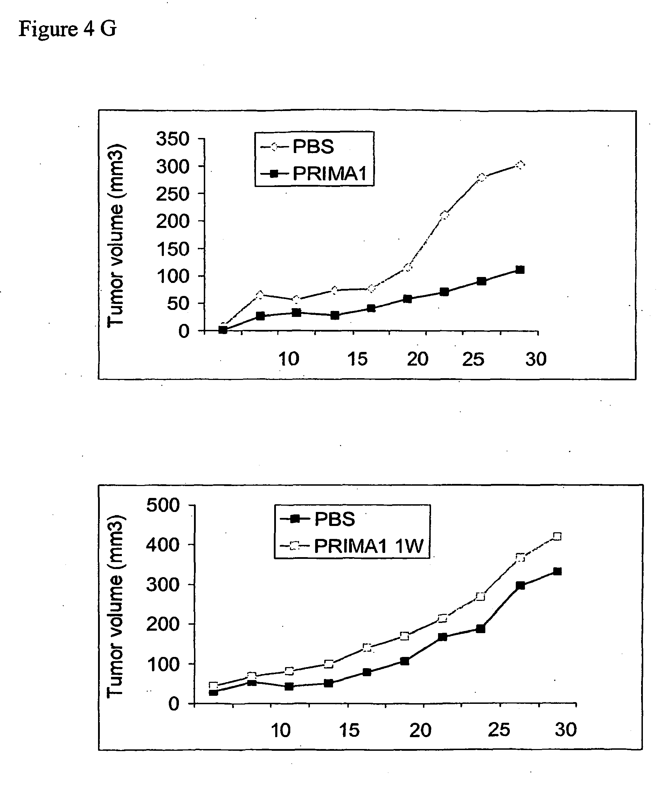

To test if restoration of an active wt p53 conformation by PRIMA-1 could also induce melanoma cell apoptosis in vivo and thereby block melanoma tumour growth, M21 and C8161 melanoma cells (1,5 x 10⁶) were injected s.c. into the back of 6-week-old C57/BL nude mice. The mice were treated with or without PRIMA-1 (100 mg/kg) for 6 d starting 6 d after tumour innoculation. The graph in Figure 4G shows mean tumour volumes calculated as described above for four mice in each group. PRIMA-1 significantly inhibited M21 melanoma growth as compared to PBS control while having no effect on C8161 melanoma growth. Given that C8161 cells have a truncated, unfunctional p53 while M21 cells carry wt p53, the results show that the effect of PRIMA-1 on melanoma growth is dependent on functional p53.

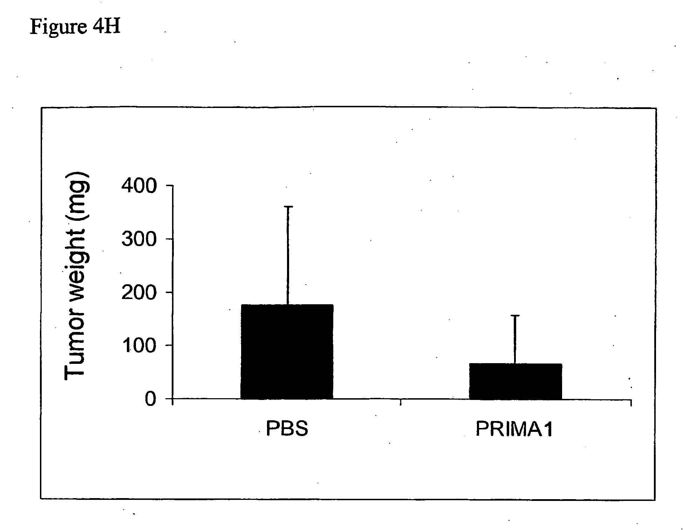

In Figure 4H, the mean of tumour wet weights plus/minus S.D. after 25 d of melanoma tumour formation with or without PRIMA-1 treatment as described in Fig. 4G are displayed. The finding that PRIMA-1 can suppress tumour growth of melanoma cells carrying wt p53 points to a novel principle for treatment of malignant melanoma by re-activating an inactive conformation of wt p53, which was kept inactive by integrin αᵥβ₃.

Based on these unexpected findings the present inventors also expect structural analogues of PRIMA-1, such as PRIMA-2 and PRIMA-3, which have been described in WO0224692, as well as further analogues of PRIMA-1 described in International Patent Application No. PCT/SE03/00206 (not published) to exhibit similar activity as PRIMA-1.

Examples of pharmaceutically acceptable addition salts for use in the pharmaceutical compositions of the present invention include those derived from mineral acids, such as hydrochlorid, hydrobromic, phosphoric, metaphosphoric, nitric and sulphuric acids, and organic acids, such as tartaric, acetic, citric, malic, lactic, fumaric, benzoic, glycolic, gluconic, succinic, and arylsulphonic acids. The pharmaceutically acceptable excipients described herein, for example, vehicles, adjuvants, carriers or diluents, are well-known to those who are skilled in the art and are readily available. The pharmaceutically acceptable carrier may be one which is chemically inert to the active compounds and which have no detrimental side effects or toxicity under the conditions of use. Pharmaceutical formulations are found e.g. in Remington: The Science and Practice of Pharmacy, 19th ed., Mack Printing Company, Easton, Pennsylvania (1995).

The composition according to the invention may be prepared for any route of administration, e.g. oral, intravenous, cutaneous or subcutaneous, nasal, intramuscular, or intraperitoneal. The precise nature of the carrier or other material will depend on the route of administration. For a parenteral administration, a parenterally acceptable aqueous solution is employed, which is pyrogen free and has requisite pH, isotonicity, and stability. Those skilled in the art are well able to prepare suitable solutions and numerous methods are described in the literature. A brief review of methods of drug delivery is also found in e.g. Langer, Science 249:1527-1533 (1990).

The dose administered to a mammal, particularly a human, in the context of the present invention should be sufficient to effect a therapeutic response in the mammal over a reasonable time frame. One skilled in the art will recognize that dosage will depend upon a variety of factors including the potency of the specific compound, the age, condition and body weight of the patient, as well as the stage/severity of the disease. The dose will also be determined by the route (administration form) timing and frequency of administration. In the case of oral administration the dosage can vary from about 0.01 mg to about 1000 mg per day of a compound of formula (I) or the corresponding amount of a pharmaceutically acceptable salt thereof.

Preferred specific examples of the compounds which can be used according to the present invention are 2,2-bis(hydroxymethyl)-1-azabicyclo[2.2.2]octan-3-one (also referred to as PRIMA-1), 9-(azabicyclo[2.2.2]octan-3-one)-6-chloro-9H-purine (also referred to as PRIMA-2), 2-(hydroxymethyl)quinuclidine-3,3-diol (also referred to as PRIMA-3), 2-(adenine-9-methylene)-3-quinuclidinone, 2-methylene-3-quinuclidinone, 2-(-2-amino-3-chloro-5-trifluoromethyl-1-methylaniline)-3-quinuclidinone, 2-(6-trifluoromethyl-4-chlorobenzimidazole-1-methylene)-3-quinuclidinone, 2-(6-methoxypurine-9-methylene)-3-quinuclidinone, 2-(8-azaadenine-9-methylene)-3-quinuclidinone, 1-azabicyclo [2.2.2]oct-3-yl benzoate, 2-(5,6-dimethyl-benzimidazole-1-methylene)-3-quinuclidinone, 2-(8-azaadenine-7-methylene)-3-quinuclidinone, 2-(7-methylene-1,3-dimethyluric acid)-3-quinuclidinone, or 2-(2,6-dichloro-9-methylenepurine)-3-quinuclidinone, or a pharmaceutically acceptable salt thereof.

According to the present invention, A more preferred group of compounds are those having a structure as defined by the following formula III 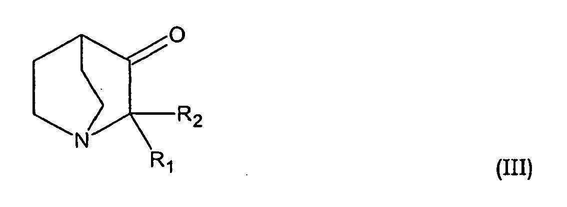 wherein
one of R₁ and R₂ is hydrogen and the other is a methylene group linked to the nitrogen atom of an amine-substituted phenyl group, to a nitrogen atom contained in the ring structure of a purine, 8-azapurine, or benzimidazol residue, and, more preferably the methylene group is linked to a nitrogen atom contained in the ring structure of a purine, 8-azapurine, or benzimidazol residue.

More preferably, one of R1 and R2 in formula II and III is hydrogen, or both R₁ and R₂ are hydroxymethyl groups.

The following compounds are believed to exhibit an activity similar or greater than that of PRIMA-1: 2-(5,6-dimethyl-benzimidazole-1-methylene)-3-quinuclidinone, 2-(8-azaadenine-7-methylene)-3-quinuclidinone, 2-(7-methylene-1,3-dimethyluric acid)-3-quinuclidinone, 2-(2,6-dichloro-9-methylenepurine)-3-quinuclidinone and 2-(6-methoxypurine-9-methylene)-3-quinuclidinone.

### General procedure for testing substances for ability of transferring wild type p53 from its inactive conformation into an active conformation

The inventive methods of testing substances are based on detection of wild type p53 in inactive conformation, either in a direct or indirect manner, as will be explained below.

In the methods of the present invention it is essential that only wild type and not mutant p53 is present. Accordingly, in any cells used in the method, only wild type p53 should be expressed, i.e. the p53 gene must not be mutated. Expression of wt p53 can readily be determined by the person skilled in the art using any suitable conventional methods, such as sequencing, mass spectroscopy, DNA-binding tests etc.

Naturally, it is also essential that the inactive form of wt p53 is present during testing. The presence of the inactive form of wt p53 can then suitably be established in a direct manner by using Pab240.

More particularly, the present inventors have found that wt p53 is inactivated under the following conditions: In melanoma cells cultured in *vitro* in a 3-dimensional environment, such as 3D collagen; in melanoma cells in tissue culture after irradiation with UV or gamma radiation, and; in melanoma cells grown *in vivo* in mice. The inventors also predict that wt p53 will take an inactive conformation in angiogenic vascular cells during angiogenesis and that the wt p53 inactive conformation is also present in additional cell types and tissues and in other cases can be induced by other means of treatment.

The method of monitoring any changes in the level of inactive wt p53 during testing is not critical according to the invention, as long as the method allows for detection of any substantial reduction in the level inactive wt p53 and a consequent induction of active p53 levels.

Based upon the above definition of the new inactive wt p53 state, and the present detailed description, the skilled person will be able to find suitable methods for detecting and/or monitoring the level of inactive wt p53 using common general knowledge. Such methods will generally be based on measurement of wt p53 conformation and/or activity state, as will be explained below in further detail.

During testing, the level of inactive wt p53 can for example be established by using a suitable conformational-specific antibody, recognising only the inactive form, such as the above-mentioned Pab240. Since the total cellular level of wt p53 is formed by the combined levels of inactive p53 and active p53, respectively, measurement of the active p53 conformation can also be used as an indirect measurement of inactive p53, i.e. an absence of active p53 indicates that p53 is instead inactive. Measurement of the active p53 conformation can also be performed using a suitable conformational-specific antibody, such as Pab 1620. If desired, the total level of wt p53 can be measured by means of a suitable antibody, which does not discriminate between the inactive and active forms of wt p53, such as DO1.

Of course, other direct or indirect parameters reflecting the activity state of wt p53 could also be used. Accordingly, the activity state of wt p53 can for example be detected by measurement of p53-bidning to DNA, by absence of expression of reporter constructs, for example using GFP or luciferase as reporters, by measurement of p53 target genes, as indicated in Figures 2C and 4D, e.g. PUMA-1, Apaf-1, PIG-3, GADD45, Mdm2, p21-CIP1, bax, bcl-2, and/or caspase-3, by using microarray-related techniques to analyse specific p53 target genes or gene expression patterns indicating p53 activity and/or by measuring the capability of wt p53 to induce apoptosis and/or growth arrest and/or the activity of molecules involved in these pathways upstream or downstream of p53, e.g. activation of caspase 9 and caspase 3 as indicated in Figure 4E.

Also, as indicated in Fig 2C and 4D, specific p53 acetylation could also be used as an indication of p53 activity. Likewise, phosphorylation of p53 is also conceivable as an indication of p53 activity.

The use of antibodies is however presently preferred for being a practical and more direct method of detecting and monitoring inactive wt p53, especially when Pab240 is used.

The general method of testing substances for the ability of transferring wt p53 from an inactive conformation into an active conformation according to the present invention will thus include the following general steps:
A. Providing cells carrying wt p53 wherein inactive wt p53 conformation is present;
B. Exposing the cells to a substance to be tested; and
C. Measuring the cellular level of inactive wt p53 conformation, for example by any of the above-mentioned methods.

The above general method can be adapted to both *in vitro* or *in vivo* testing by exposing the cells in step B either in *vivo* or in *vitro* to the substance to be tested, as will be readily understood hereinafter.

Step C is typically performed both before and after exposing the cell in step B to a substance to be tested.

### In vitro testing of substances for ability of transferring wild type p53 from its inactive conformation into an active conformation

In the case of in *vitro* testing for substances capable of reverting an inactive p53 conformation to an active conformation, cells carrying wt p53 can be grown in a 3-dimensional environment and the conformation and/or activity state of p53 measured, for example by any of the methods mentioned above, with and without, respectively, the addition of a substance for testing. A substance is considered functional if it is found to be capable of changing the conformation and/or activity of inactive wt p53. Alternatively, cells with wt p53 could be irradiated in order to induce the inactive wt p53 conformation, exposed to a substance to be tested, and thereafter analysed for p53 conformation and/or activity. Alternatively, a cell naturally displaying an inactive wt p53 conformation could be utilized or an inactive wt p53 conformation could be induced by other means, the cell thereafter being exposed to a substance to be tested, and analysed for inactive p53 state.

A tissue, an organ, or a part thereof, from a human or from an animal could also be cultured, treated and used for the inventive testing as described for cells *in vitro* above. Although such models sometimes are referred to as *ex-vivo* models, when used in the inventive method of testing of substances herein, such method is considered to be a method of *in-vitro* testing.

Alternatively, *in-vitro* testing of substances could be performed in models of angiogenesis. In vitro examples include, but are not limited to, tube forming assays of endothelial cells, outgrowth of blood vessels from stem cells, outgrowth of blood vessels from an isolated vessel originating from an animal or human, or analysis of endothelial cells in tissue culture.

An *in-vitro* testing procedure could thus for example include the following general steps:
A. Providing cells carrying wt p53 *in vitro,* and inducing inactive wt p53 conformation if not already present;
B. Exposing the cells to a substance to be tested; and
C. Measuring the cellular level of inactive wt p53 conformation, directly or indirectly for example by any of the above-mentioned methods.

Step C is typically performed both before and after exposing the cell in step B to a substance to be tested.

### In vivo testing of substances for ability of transferring wild type p53 from its inactive conformation into an active conformation

Likewise, wt p53 conformation and/or activity could be assessed *in vivo*. For example, melanoma cells xenografts could be used *in vivo* to test substances for their capacity of reverting an inactive p53 conformation to active p53. In this case, an animal carrying such tumour xenograft would be treated with a substance to be tested, and the effect of this substance on wt p53 conformation and activity measured as described above. Alternatively, testing of substances could be carried out in animal melanoma models, where melanoma is occurring naturally or is provoked within the animal by treatment e.g. by UV-radiation and/or by carcinogenic chemicals and/or by genetic modifications. Administration of test substance to the animal can be performed i.v., i.p., s.c., intratumoural, or otherwise.

Alternatively, in-vivo testing of substances could be performed in models of angiogenesis. Examples of *in-vivo* models include, but are not limited to, stimulation of angiogenesis in the chick embryo chorioallantoic membrane, stimulation of angiogenesis by a transplant of extracellular matrix or comparable substances onto mice or onto another animal, stimulation of angiogenesis in the cornea by an angiogenic compound, stimulation of angiogenesis in the retina by hypoxia, analysis of developmental angiogenesis in the retina or elsewhere, and induction of angiogenesis by a tumour xenograft or another type of xenograft. Measurement of angiogenesis is performed by a quantification of the number of blood vessels within the tissue and/or by quantification of vascular cell markers and/or by measurement of vascular cell apoptosis and/or of vascular cell proliferation and/or by similar means.

An in-vivo testing procedure could for example comprise the following general steps:
A. Providing an animal expressing wt p53 in a tissue thereof, wherein inactive wt p53 conformation is present;
B. Exposing cells of the tissue of the animal *in vivo* to a substance to be tested; and
C. Measuring the cellular level of inactive wt p53 conformation, directly or indirectly, for example by any of the above-mentioned methods.

Step C is typically performed both before and after exposing the cell in step B to a substance to be tested.

Alternatively, and as illustrated in Figure 4G positive testing of a compound can also be established by comparing the effect of the tested substance in cells or tissues carrying functional p53 to the effect on cells or tissues with no or non-functional p53. Obviously, the observable effect can for example relate to a reduced tumour volume or weight, or number of tumour cells or induction of apoptosis or indications thereof, e.g. as described in Figure 4. Accordingly, in the above in *vivo* and *in vitro* methods, instead of step C, an alternative step C' can be used comprising comparing the effect of the tested substance on the cells or tissue (carrying functional p53) in step B to the effect on cells or tissues with no or non-functional p53.

Alternatively, testing of a compound can also be tested by comparing the effect of the tested substance on other physiological or pathological mechanisms where wt p53 conformation could play an active role for the regulation, e.g. in angiogenesis as motivated above. In such cases, the observable effects could be the effect of the tested substance on the particular physiological or pathological events, e.g. the amount of newly formed blood vessels formed by angiogenesis and/ or the amount of apoptosis or indications thereof in vascular cells.

The animals are typically sacrificed after the above-described in-vivo testing of substances.

## Claims

1. Use of a compound capable of transferring wild type p53 from an inactive conformation thereof, which conformation is reactive to Pab 240 and not to Pab 1620, into an active conformation capable of inducing apoptosis, which compound is selected from compounds having a structure according to the formula I 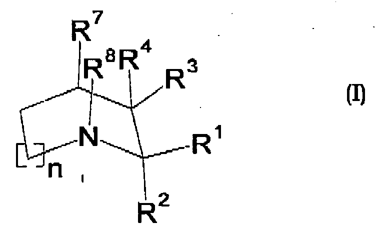 wherein
n is 0,1 or 2;
R¹ and R² are the same or different and are selected from -H, -CH₂-R⁵, -CH₂-O-R⁵, -CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ and -CH₂-O-CO-NHR⁵ ; or R¹ and R² are together =CH₂;
R³ and R⁴ are the same or different and are selected from -H, -OH, -SH, -NH₂, -NHR⁵ and -O-CO-C₆H₅; or R³ and R⁴ together are =O, =S, =NH or =NR⁵;
R⁵ represents the same or different groups selected from H, substituted or non-substituted C1 to C10 alkyl, C2 to C 10 alkenyl, C2 to C 10 alkynyl, substituted or non-substituted C3 to C12 cycloalkyl, substituted or non-substituted benzyl groups, substituted or non-substituted aryl or mono-, bi-, tricyclic unsubstituted or substituted heteroaromatic ring(s) with one or more heteroatoms and non-aromatic heterocycles wherein
the substituents of the substituted groups are selected from C1 to C 10 alkyl, C2 to C10 alkenyl, C2 to C10 alkynyl, halogen, substituted or non-substituted aryl, substituted or non-substituted heteroaromatic compounds, non-aromatic heterocycles, C1 to C10 alkyloxy, C1 to C 10 alkylamino, C2 to C10 alkenylamino, C2 to C10 alkynylamino, COR⁶, CONR⁶ and COOR⁶;
R⁶ is selected from H, unsubstituted or substituted C1 to C 10 alkyl, C2 to C10 alkenyl or alkynyl, benzyl, aryl, unsubstituted or substituted heteroaromatic rings with one or more heteroatoms and non-aromatic heterocycles;
R⁷ and R⁸ together form a bridging CH₂-CH₂ moiety; or R⁷ and R⁸ are both hydrogen;
or a pharmaceutically acceptable salt or prodrug thereof,
for the preparation of a medicament for use in treating malignant melanoma.

2. The use of claim 1, wherein the compound is selected from compounds having the following formula (II) 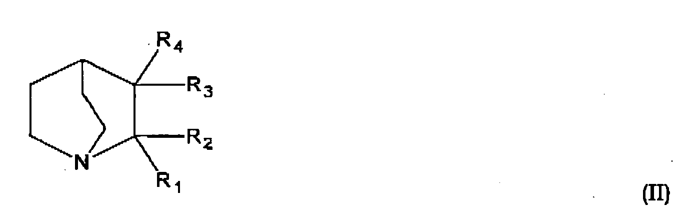 wherein:
R₁ and R₂ are independently selected from hydrogen, hydroxymethyl, or a methylene group linked to the nitrogen atom of an amine-substituted phenyl group, to a nitrogen atom contained in the ring structure of a purine, 8-azapurine, or benzimidazol residue, or R₁ and R₂ may together represent a double bonded methylene group, and;
R₃ and R₄ are independently selected from hydrogen, hydroxyl, and benzoyloxy, or R₃ and R₄ may together represent an oxygen atom being double bonded, with the proviso that when either of R₃ and R₄ is a benzoyloxy group, both R₁ and R₂ are hydrogen, or a pharmaceutically acceptable salt or prodrug thereof.

3. The use of claim 2, wherein the compound is selected from 2,2-bis(hydroxymethyl)-1-azabicyclo[2.2.2]octan-3-one, 9-(azabicyclo[2.2.2]octan-3-one)-6-chloro-9H-purine, 2-(hydroxymethyl)quinuclidine-3,3-diol, 2-(adenine-9-methylene)-3-quinuclidinone, 2-methylene-3-quinuclidinone, 2-(2-amino-3-chloro-5-trifluoromethyl-1-methylaniline)-3-quinuclidinone, 2-(6-trifluoromethyl-4-chlorobenzimidazole-l-methylene)-3-quinuclidinone, 2-(6-methoxypurine-9-methylene)-3-quinuclidinone, 2-(8-azaadenine-9-methylene)-3-quinuclidinone, 1-azabicyclo [2.2.2]oct-3-yl benzoate, 2-(5,6-dimethyl-benzimidazole-1-methylene)-3-quinuclidinone, 2-(8-azaadenine-7-methylene)-3-quinuclidinone, 2-(7-methylene-1,3-dimethyluric acid)-3-quinuelidinone, or 2-(2,6-dichloro-9-methylenepurine)-3-quinuclidinone, or a pharmaceutically acceptable salt thereof.

4. Use of a compound capable of transferring wild type p53 from an inactive conformation thereof, which conformation is reactive to Pab 240 and not to Pab 1620, into an active conformation capable of inducing apoptosis, which compound is selected from compounds having a structure according to the formula I 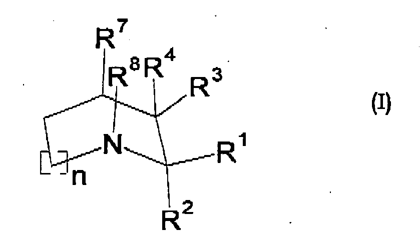 wherein
n is 0,1 or 2;
R¹ and R² are the same or different and are selected from -H, -CH₂-R⁵, -CH₂-O-R⁵, -CH₂-S-R⁵_{,} -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ and -CH₂-O-CO-NHR⁵ ; or R¹ and R² are together =CH₂;
R³ and R⁴ are the same or different and are selected from -H, -OH, -SH, -NH₂, -NHR⁵ and -O-CO-C₆H₅; or R³ and R⁴ together are =O, =S, =NH or =NR⁵;
R5 represents the same or different groups selected from H, substituted or non-substituted C1 to C10 alkyl, C2 to C10 alkenyl, C2 to C10 alkynyl, substituted or non-substituted C3 to C12 cycloalkyl, substituted or non-substituted benzyl groups, substituted or non-substituted aryl or mono-, bi-, tricyclic unsubstituted or substituted heteroaromatic ring(s) with one or more heteroatoms and non-aromatic heterocycles wherein
the substituents of the substituted groups are selected from C1 to C10 alkyl, C2 to C10 alkenyl, C2 to C10 alkynyl, halogen, substituted or non-substituted aryl, substituted or non-substituted heteroaromatic compounds, non-aromatic heterocycles, C1 to C10 alkyloxy, C1 to C10 alkylamino, C2 to C10 alkenylamino, C2 to C10 alkynylamino, COR⁶, CONR⁶ and COOR⁶;
R⁶ is selected from H, unsubstituted or substituted C1 to C10 alkyl, C2 to C10 alkenyl or alkynyl, benzyl, aryl, unsubstituted or substituted heteroaromatic rings with one or more heteroatoms and non-aromatic heterocycles;
R⁷ and R⁸ together form a bridging CH₂-CH₂ moiety; or R⁷ and R⁸ are both hydrogen;
or a pharmaceutically acceptable salt or prodrug thereof,
with the proviso that when
n is 1;
R³ and R⁴ are selected from -O-CO-C₆H₅ and =O; and
R⁷ and R⁸ together form a bridging CH₂-CH₂ moiety;
R¹ and R² are not both -H and do not together form a double-bonded methylene group =CH₂ or a single bonded methylene group linked to the nitrogen atom of an amine-substituted phenyl group, or to a nitrogen atom contained in the ring structure of a purine, 8-azapurine, or benzimidazol residue;
for the preparation of a medicament for use in treating malignant melanoma and/or a pathological condition involving undesired angiogenesis.

5. The use of claim 4, wherein the compound is selected from compounds having the following formula (II) 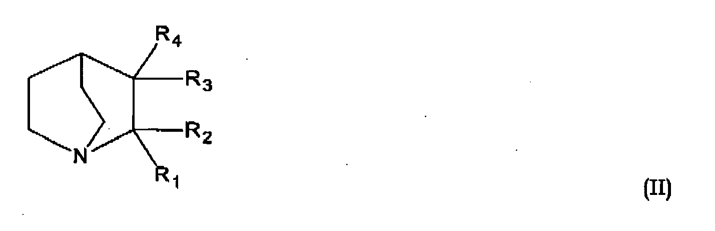 wherein:
R₁ and R₂ are independently selected from hydrogen, hydroxymethyl, or a methylene group linked to the nitrogen atom of an amine-substituted phenyl group, to a nitrogen atom contained in the ring structure of a purine, 8-azapurine, or benzimidazol residue, or R₁ and R₂ may together represent a double bonded methylene group, and;
R₃ and R₄ are independently selected from hydrogen, hydroxyl, and benzoyloxy, or R₃ and R₄ may together represent an oxygen atom being double bonded, with the proviso that when either of R₃ and R₄ is a benzoyloxy group, both R₁ and R₂ are hydrogen, or a pharmaceutically acceptable salt or prodrug thereof.

6. The use of claim 5, wherein the compound is selected from 2,2-bis(hydroxymethyl)-1-azabicyclo[2.2.2]octan-3-one, 9-(azabicyclo[2.2.2]octan-3-one)-6-chloro-9H-purine, 2-(hydroxymethyl)quinuclidine-3,3-diol, or a pharmaceutically acceptable salt thereof.

7. The use of any of the claims 1-6 together with a pharmaceutically acceptable carrier, diluent and/ or excipient.

8. A compound capable of transferring wild type p53 from an inactive conformation thereof, which conformation is reactive to Pab 240 and not to Pab 1620, into an active conformation capable of inducing apoptosis, which compound is selected from compounds having a structure according to the formula I 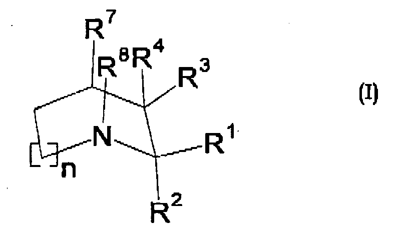 wherein
n is 0,1 or 2;
R¹ and R² are the same or different and are selected from -H, -CH₂-R⁵, -CH₂-O-R₅, -CH₂-S-R⁵, -CH₂-NH-R⁵, -CO-O-R⁵, -CO-NH-R⁵, -CH₂-NH-CO-R⁵, -CH₂-O,CO-R⁵, -CH₂-NH-CO-NHR⁵, -CH₂-NH-CO-OR⁵, -CH₂-NH-CS-NHR⁵ and -CH₂-O-CO-NHR⁵ ; or R¹ and R² are together =CH₂;
R³ and R⁴ are the same or different and are selected from -H, -OH, -SH, -NH₂, -NHR⁵ and -O-CO-C₆H₅; or R³ and R⁴ together are =O, =S, =NH or =NR⁵;
R⁵ represents the same or different groups selected from H, substituted or non-substituted C1 to C10 alkyl, C2 to C10 alkenyl, C2 to C10 alkynyl, substituted or non-substituted C3 to C12 cycloalkyl, substituted or non-substituted benzyl groups, substituted or non-substituted aryl or mono-, bi-, tricyclic unsubstituted or substituted heteroaromatic ring(s) with one or more heteroatoms and non-aromatic heterocycles wherein
the substituents of the substituted groups are selected from C 1 to C10 alkyl, C2 to C 10 alkenyl, C2 to C10 alkynyl, halogen, substituted or non-substituted aryl, substituted or non-substituted heteroaromatic compounds, non-aromatic heterocycles, C1 to C10 alkyloxy, C1 to C10 alkylamino, C2 to C10 alkenylamino, C2 to C10 alkynylamino, COR⁶, CONR⁶ and COOR⁶;
R⁶ is selected from H, unsubstituted or substituted C1 to C10 alkyl, C2 to C10 alkenyl or alkynyl, benzyl, aryl, unsubstituted or substituted heteroaromatic rings with one or more heteroatoms and non-aromatic heterocycles;
R⁷ and R⁸ together form a bridging CH₂-CH₂ moiety; or R⁷ and R⁸ are both hydrogen;
or a pharmaceutically acceptable salt or prodrug thereof,
for use in treating malignant melanoma.

9. The compound of claim 8, wherein the compound is selected from compounds having the following formula (II) 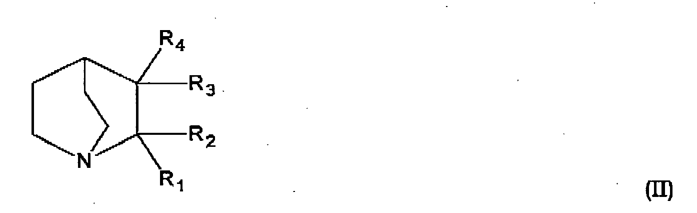 wherein:
R₁ and R₂ are independently selected from hydrogen, hydroxymethyl, or a methylene group linked to the nitrogen atom of an amine-substituted phenyl group, to a nitrogen atom contained in the ring structure of a purine, 8-azapurine, or benzimidazol residue, or R₁ and R₂ may together represent a double bonded methylene group, and;
R₃ and R₄ are independently selected from hydrogen, hydroxyl, and benzoyloxy, or R₃ and R₄ may together represent an oxygen atom being double bonded, with the proviso that when either of R₃ and R₄ is a benzoyloxy, group, both R₁ and R₂ are hydrogen, or a pharmaceutically acceptable salt or prodrug thereof.

10. The compound of claim 9, wherein the compound is selected from 2,2-bis(hydroxymethyl)-1-azabicyclo[2.2.2]octan-3-one, 9-(azabicyclo[2.2.2]octan-3-one)-6-chloro-9H-purine, 2-(hydroxymethyl)quinuclidine-3,3-diol, 2-(adenine-9-methylene)-3-quinuclidinone, 2-methylene-3-quinuclidinone, 2-(2-amino-3-chloro-5-trifluoromethyl-1-methylaniline)-3-quinuclidinone, 2-(6-trifluoromethyl-4-chlorobenzimidazole-1-methylene)-3-quinuclidinone, 2-(6-methoxypurine-9-methylene)-3-quinuclidinone, 2-(8-azaadenine-9-methylene)-3-quinuclidinone, 1-azabicyclo [2.2.2]oct-3-yl benzoate, 2-(5,6-dimethyl-benzimidazole-1-methylene)-3-quinuclidinone, 2-(8-azaadenine-7-methylene)-3-quinuclidinone, 2-(7-methylene-1,3-dimethyluric acid)-3-quinuclidinone, or 2-(2,6-dichloro-9-methylenepurine)-3-quinuclidinone, or a pharmaceutically acceptable salt thereof.

11. A compound capable of transferring wild type p53 from an inactive conformation thereof, which conformation is reactive to Pab 240 and not to Pab 1620, into an active conformation capable of inducing apoptosis, which compound is selected from compounds having a structure according to the formula I 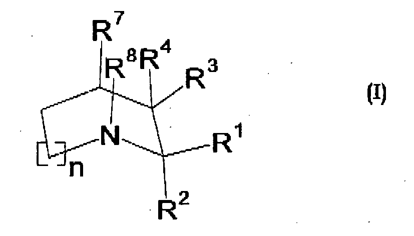 wherein
n is 0,1 or 2;
R¹ and R² are the same or different and are selected from -H, -CH₂-R⁵, -CH₂-O-R⁵, -CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ and -CH₂-O-CO-NHR⁵ ; or R¹ and R² are together =CH₂;
R³ and R⁴ are the same or different and are selected from, -H, -OH, -SH, -NH₂, -NHR⁵ and -O-CO-C₆H₅; or R³ and R⁴ together are =O, =S, =NH or =NR⁵;
R⁵ represents the same or different groups selected from H, substituted or non-substituted C1 to C 10 alkyl, C2 to C10 alkenyl, C2 to C10 alkynyl, substituted or non-substituted C3 to C12 cycloalkyl, substituted or non-substituted benzyl groups, substituted or non-substituted aryl or mono-, bi-, tricyclic unsubstituted or substituted heteroaromatic ring(s) with one or more heteroatoms and non-aromatic heterocycles wherein
the substituents of the substituted groups are selected from C1 to C10 alkyl, C2 to C10 alkenyl, C2 to C 10 alkynyl, halogen, substituted or non-substituted aryl, substituted or non-substituted heteroaromatic compounds, non-aromatic heterocycles, C1 to C 10 alkyloxy, C1 to C 10 alkylamino, C2 to C 10 alkenylamino, C2 to C10 alkynylamino, COR⁶, CONR⁶ and COOR6;
R6 is selected from H, unsubstituted or substituted C1 to C 10 alkyl, C2 to C10 alkenyl or alkynyl, benzyl, aryl, unsubstituted or substituted heteroaromatic rings with one or more heteroatoms and non-aromatic heterocycles;
R⁷ and R⁸ together form a bridging CH₂-CH₂ moiety; or R⁷ and R⁸ are both hydrogen;
or a pharmaceutically acceptable salt or prodrug thereof,
with the proviso that when
n is 1;
R³ and R⁴ are selected from -O-CO-C₆H₅ and =O; and
R⁷ and R⁸ together form a bridging CH₂-CH₂ moiety;
R¹ and R² are not both -H and do not together form a double-bonded methylene group =CH₂ or a single bonded methylene group linked to the nitrogen atom of an amine-substituted phenyl group, or to a nitrogen atom contained in the ring structure of a purine, 8-azapurine, or benzimidazol residue;
for use in treating malignant melanoma and/or a pathological condition involving undesired angiogenesis.

12. The compound of claim 11, wherein the compound is selected from compounds having the following formula (II) 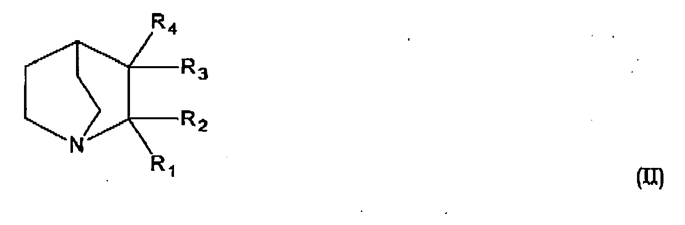 wherein:
R₁ and R₂ are independently selected from hydrogen, hydroxymethyl, or a methylene group linked to the nitrogen atom of an amine-substituted phenyl group, to a nitrogen atom contained in the ring structure of a purine, 8-azapurine, or benzimidazol residue, or R₁ and R₂ may together represent a double bonded methylene group, and;
R₃ and R₄ are independently selected from hydrogen, hydroxyl, and benzoyloxy, or R₃ and R₄ may together represent an oxygen atom being double bonded, with the proviso that when either of R₃ and R₄ is a benzoyloxy group, both R₁ and R₂ are hydrogen, or a pharmaceutically acceptable salt or prodrug thereof.

13. The compound of claim 12, wherein the compound is selected from 2,2-bis(hydroxymethyl)-1-azabicyclo[2.2.2]octan-3-one, 9-(azabicyclo[2.2.2]octan-3-one)-6-chloro-9H-purine, 2-(hydroxymethyl)quinuclidine-3,3-diol, or a pharmaceutically acceptable salt thereof.

14. The compound of any of the claims 8-13 together with a pharmaceutically acceptable carrier, diluent and/or excipient.

15. Method of testing compounds for the ability of transferring wild type p53 from an inactive conformation into an active conformation comprising the steps:
A. Providing cells carrying only wt and not mutant p53, in which cells inactive wt p53 conformation is present;
B. Exposing the cells *in vitro* to a substance to be tested; and
C. Measuring the cellular inactive wt p53 conformation.

16. The method of claim 15, wherein instead of step C an alternative step C' is used comprising comparing the effect of the tested substance on the cells (carrying functional p53) in step B to the effect on cells or tissues with no or non-functional p53.

17. The method of claim 15 or 16, wherein integrin αᵥβ₃ is present in the cells.

18. The method of claim 15-17, wherein the Pab 240 is used for detecting wt p53 in its inactive conformation.

19. The method of any of the claims 15-18, wherein a compound having a structure according to the formula I 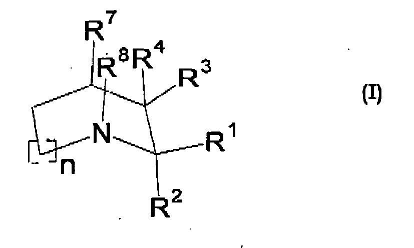 wherein
n is 0,1 or 2;
R¹ and R² are the same or different and are selected from -H, -CH₂-R⁵, -CH₂-O-R⁵, -CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ and -CH₂-O-CO-NHR⁵ ; or R¹ and R² are together =CH₂;
R³ and R⁴ are the same or different and are selected from -H, -OH, -SH, -NH₂, -NHR⁵ and -O-CO-C₆H₅; or R³ and R⁴ together are =O, =S, =NH or =NR⁵;
R⁵ represents the same or different groups selected from H, substituted or non-substituted C1 to C10 alkyl, C2 to C 10 alkenyl, C2 to C10 alkenyl, substituted or non-substituted C3 to C12 cycloalkyl, substituted or non-substituted benzyl groups, substituted or non-substituted aryl or mono-, bi-, tricyclic unsubstituted or substituted heteroaromatic ring(s) with one or more heteroatoms and non-aromatic heterocycles wherein
the substituents of the substituted groups are selected from C1 to C10 alkyl, C2 to C10 alkenyl, C2 to C10 alkynyl, halogen, substituted or non-substituted aryl, substituted or non-substituted heteroaromatic compounds, non-aromatic heterocycles, C 1 to C10 alkyloxy, C1 to C 10 alkylamino, C2 to C10 alkenylamino, C2 to C10 alkynylamino, COR⁶, CONR⁶ and COOR⁶;
R⁶ is selected from H, unsubstituted or substituted C1 to C 10 alkyl, C2 to C10 alkenyl or alkynyl, benzyl, aryl, unsubstituted or substituted heteroaromatic rings with one or more heteroatoms and non-aromatic heterocycles;
R⁷ and R⁸ together form a bridging CH₂-CH₂ moiety; or R⁷ and R⁸ are both hydrogen;
or a pharmaceutically acceptable salt or prodrug thereof is tested.

20. The method of any of the claims 15-19, wherein the cells in step B are exposed *in vivo* in an animal to the substance to be tested, and the animal subsequently sacrificed.

## Patentansprüche

1. Verwendung einer Verbindung, welche Wildtyp p53 aus einer inaktiven Konformation davon, wobei die Konformation mit Pab 240 und nicht mit Pab 1620 reaktiv ist, in eine aktive Konformation überführen kann, welche Apoptose herbeiführen kann, wobei die Verbindung ausgewählt ist aus Verbindungen mit einer Struktur gemäß der Formel I 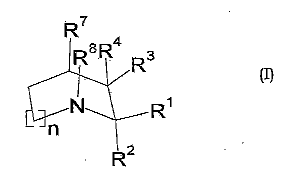 wobei
n 0, 1 oder 2 ist;
R¹ und R² gleich oder verschieden sind und ausgewählt sind aus -H, -CH₂-R⁵, -CH₂-O-R⁵, -CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ und -CH₂-O-CO-NHR⁵, oder R¹ und R² zusammen =CH₂ sind;
R³ und R⁴ gleich oder verschieden sind und ausgewählt sind aus -H, -OH, -SH, -NH₂, -NHR⁵ und -O-CO-C₆H₅, oder R³ und R⁴ zusammen =O, =S, =NH oder =NR⁵ sind;
R⁵ gleiche oder verschiedene Reste darstellt, ausgewählt aus H, substituiertem oder nicht substituiertem C1 bis C10 Alkyl, C2 bis C10 Alkenyl, C2 bis C10 Alkinyl, substituiertem oder nicht substituiertem C3 bis C12 Cycloalkyl, substituierten oder nicht substituierten Benzylresten, substituiertem oder nicht substituiertem Aryl oder mono-, bi-, tricyclischen(m) unsubstituierten(m) oder substituierten(m) heteroaromatischen(m) Ring(en) mit einem oder mehreren Heteroatomen und nicht aromatischen Heterocyclen, wobei
die Substituenten der substituierten Reste ausgewählt sind aus C1 bis C10 Alkyl, C2 bis C10 Alkenyl, C2 bis C10 Alkinyl, Halogen, substituiertem oder nicht substituiertem Aryl, substituierten oder nicht substituierten heteroaromatischen Verbindungen, nicht aromatischen Heterocyclen, C1 bis C10 Alkyloxy, C1 bis C10 Alkylamin, C2 bis C10 Alkenylamino, C2 bis C10 Alkinylamino, COR⁶, CONR⁶ und COOR⁶;
R⁶ ausgewählt ist aus H, unsubstituiertem oder substituiertem C1 bis C10 Alkyl, C2 bis C10 Alkenyl oder Alkinyl, Benzyl, Aryl, unsubstituierten oder substituierten heteroaromatischen Ringen mit einem oder mehreren Heteroatomen und nicht aromatischen Heterocyclen;
R⁷ und R⁸ zusammen eine verbrückende CH₂-CH₂-Einheit bilden, oder R⁷ und R⁸ beide Wasserstoff sind;
oder eines pharmazeutisch verträglichen Salzes oder Prodrugs davon;
zur Herstellung eines Medikaments zur Verwendung bei der Behandlung von malignem Melanom.

2. Verwendung gemäß Anspruch 1, wobei die Verbindung ausgewählt ist aus Verbindungen der folgenden Formel (II) 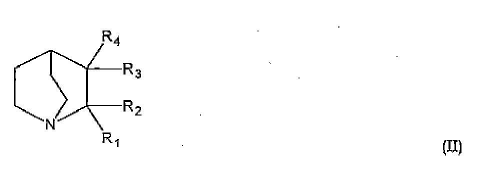 wobei:
R₁ und R₂ unabhängig ausgewählt sind aus Wasserstoff, Hydroxymethyl oder einem Methylenrest, welcher verbunden ist mit dem Stickstoffatom eines Amin-substituierten Phenylrests, mit dem Stickstoffatom, welches in der Ringstruktur eines Purin-, 8-Azapurin- oder Benzimidazolrests enthalten ist, oder R₁ und R₂ zusammen einen doppelt gebundenen Methylenrest darstellen können; und
R₃ und R₄ unabhängig ausgewählt sind aus Wasserstoff, Hydroxyl und Benzoyloxy, oder R₃ und R₄ zusammen ein Sauerstoffatom, welches doppelt gebunden ist, darstellen können, mit der Maßgabe, dass wenn einer von R₃ und R₄ ein Benzoyloxyrest ist, sowohl R₁ als auch R₂ Wasserstoff ist, oder einem pharmazeutisch verträglichen Salz oder Prodrug davon.

3. Verwendung gemäß Anspruch 2, wobei die Verbindung ausgewählt ist aus 2,2-Bis(hydroxymethyl)-1-azabicyclo[2.2.2]octan-3-on, 9-(Azabicyclo[2.2.2]octan-3-on)-6-chlor-9H-purin, 2-(Hydroxymethyl)chinuclidin-3,3-diol, 2-(Adenin-9-methylen)-3-chinuclidinon, 2-Methylen-3-chinuclidinon, 2-(2-Amino-1o-3-chlor-5-trifluormethyl-1-methylanilin)-3-chinuclidinon, 2-(6-Trifluormethyl-4-chlorbenzimidazol-1-methylen)-3-chinuclidinon, 2-(6-Methoxypurin-9-methylen)-3-chinuclidinon, 2-(8-Azaadenin-9-methylen)-3-chinuclidinon, 1-Azabicyclo[2.2.2]oct-3-ylbenzoat, 2-(5,6-Dimethylbenz-imidazol-1-methylen)-3-chinuclidinon, 2-(8-Azaadenin-7-methylen)-3-chinuclidinon, 2-(7-Methylen-1,3-dimethylharnsäure)-3-chinuclidinon oder 2-(2,6-Dichlor-9-methylen-purin)-3-chinuclidinon, oder einem pharmazeutisch verträglichen Salz davon.

4. Verwendung einer Verbindung, welche Wildtyp p53 aus einer inaktiven Konformation davon, wobei die Konformation mit Pab 240 und nicht mit Pab 1620 reaktiv ist, in eine aktive Konformation überführen kann, welche Apoptose herbeiführen kann, wobei die Verbindung ausgewählt ist aus Verbindungen mit einer Struktur gemäß der Formel I 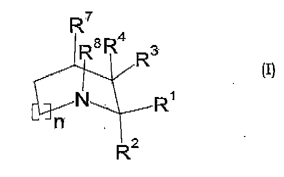 wobei
n 0, 1 oder 2 ist;
R¹ und R² gleich oder verschieden sind und ausgewählt sind aus -H, -CH₂-R⁵, -CH₂-O-R⁵, -CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH²-NH-CO-OR⁵, -CH₂-NH-CS-NHR⁵ und -CH₂-O-CO-NHR⁵, oder R¹ und R² zusammen =CH₂ sind;
R³ und R⁴ gleich oder verschieden sind und ausgewählt sind aus -H, -OH, -SH, -NH₂, -NHR⁵ und -O-CO-C₆H₅, oder R³ und R⁴ zusammen =O, =S, =NH oder =NR⁵ sind;
R⁵ gleiche oder verschiedene Reste darstellt, ausgewählt aus H, substituiertem oder nicht substituiertem C1 bis C10 Alkyl, C2 bis C10 Alkenyl, C2 bis C10 Alkinyl, substituiertem oder nicht substituiertem C3 bis C12 Cycloalkyl, substituierten oder nicht substituierten Benzylresten, substituiertem oder nicht substituiertem Aryl oder mono-, bi-, tricyclischen(m) unsubstituierten(m) oder substituierten(m) heteroaromatischen(m) Ring(en) mit einem oder mehreren Heteroatomen und nicht aromatischen Heterocyclen, wobei
die Substituenten der substituierten Reste ausgewählt sind aus C1 bis C10 Alkyl, C2 bis C10 Alkenyl, C2 bis C10 Alkinyl, Halogen, substituiertem oder nicht substituiertem Aryl, substituierten oder nicht substituierten heteroaromatischen Verbindungen, nicht aromatischen Heterocyclen, C1 bis C10 Alkyloxy, C1 bis C10 Alkylamino, C2 bis C10 Alkenylamino, C2 bis C10 Alkinylamino, COR⁶, CONR⁶ und COOR⁶;
R⁶ ausgewählt ist aus H, unsubstituiertem oder substituiertem C1 bis C10 Alkyl, C2 bis C10 Alkenyl oder Alkinyl, Benzyl, Aryl, unsubstituierten oder substituierten heteroaromatischen Ringen mit einem oder mehreren Heteroatomen und nicht aromatischen Heterocyclen;
R⁷ und R⁸ zusammen eine verbrückende CH₂-CH₂-Einheit bilden, oder R⁷ und R⁸ beide Wasserstoff sind;
oder eines pharmazeutisch verträglichen Salzes oder Prodrugs davon,
mit der Maßgabe, dass wenn
n 1 ist;
R³ und R⁴ ausgewählt sind aus -O-CO-C₆H₅ und =O; und
R⁷ und R⁸ zusammen eine verbrückende CH₂-CH₂-Einheit bilden;
R¹ und R² nicht beide -H sind und nicht zusammen einen doppelt gebundenen Methylenrest =CH₂ oder einen einfach gebundenen Methylenrest bilden, welcher verbunden ist mit dem Stickstoffatom eines Amin-substituierten Phenylrests oder mit dem Stickstoffatom, welches in der Ringstruktur eines Purin-, 8-Azapurin- oder Benzimidazolrests enthalten ist;
zur Herstellung eines Medikaments zur Verwendung bei der Behandlung von malignem Melanom und/oder eines pathologischen Zustands, welcher unerwünschte Angiogenese einschließt.

5. Verwendung gemäß Anspruch 4, wobei die Verbindung ausgewählt ist aus Verbindungen der folgenden Formel (II) 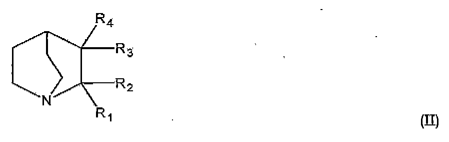 wobei:
R₁ und R₂ unabhängig ausgewählt sind aus Wasserstoff, Hydroxymethyl oder einem Methylenrest, welcher verbunden ist mit dem Stickstoffatom eines Amin-substituierten Phenylrests, mit dem Stickstoffatom, welches in der Ringstruktur eines Purin-, 8-Azapurin- oder Benzimidazolrests enthalten ist, oder R₁ und R₂ zusammen einen doppelt gebundenen Methylenrest darstellen können, und
R₃ und R₄ unabhängig ausgewählt sind aus Wasserstoff, Hydroxyl und Benzoyloxy, oder R₃ und R₄ zusammen ein Sauerstoffatom, welches doppelt gebunden ist, darstellen können, mit der Maßgabe, dass wenn einer von R₃ und R₄ ein Benzyloxyrest ist, sowohl R₁ als auch R₂ Wasserstoff ist, oder einem pharmazeutisch verträglichen Salz oder Prodrug davon.

6. Verwendung gemäß Anspruch 5, wobei die Verbindung ausgewählt ist aus 2,2-Bis(hydroxymethyl)-1-azabicyclo[2.2.2]octan-3-on, 9-(Azabicyclo[2.2.2]octan-3-on)-6-chlor-9H-purin, 2-(Hydroxymethyl)chinuclidin-3,3-diol, oder einem pharmazeutisch verträglichen Salz davon.

7. Verwendung gemäß einem der Ansprüche 1 bis 6, zusammen mit einem pharmazeutisch verträglichen Träger, Verdünnungsmittel und/oder Exzipienten.

8. Verbindung, welche Wildtyp p53 aus einer inaktiven Konformation davon, wobei die Konformation mit Pab 240 und nicht mit Pab 1620 reaktiv ist, in eine aktive Konformation überführen kann, welche Apoptose herbeiführen kann, wobei die Verbindung ausgewählt ist aus Verbindungen mit einer Struktur gemäß der Formel I 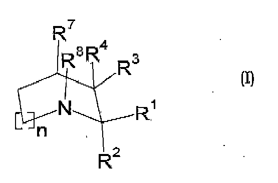 wobei
n 0, 1 oder 2 ist;
R¹ und R² gleich oder verschieden sind und ausgewählt sind aus -H, -CH₂-R⁵, -CH₂-O-R⁵, -CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ und -CH₂-O-CO-NHR⁵, oder R¹ und R² zusammen =CH₂ sind;
R³ und R⁴ gleich oder verschieden sind und ausgewählt sind aus -H, -OH, -SH, -NH₂, -NHR⁵ und -O-CO-C₆H₅, oder R³ und R⁴ zusammen =O, =S, =NH oder =NR⁵ sind;
R⁵ gleiche oder verschiedene Reste darstellt, ausgewählt aus H, substituiertem oder nicht substituiertem C1 bis C10 Alkyl, C2 bis C10 Alkenyl, C2 bis C10 Alkinyl, substituiertem oder nicht substituiertem C3 bis C12 Cycloalkyl, substituierten oder nicht substituierten Benzylresten, substituiertem oder nicht substituiertem Aryl oder mono-, bi-, tricyclischen(m) unsubstituierten(m) oder substituierten(m) heteroaromatischen(m) Ring(en) mit einem oder mehreren Heteroatomen und nicht aromatischen Heterocyclen, wobei
die Substituenten der substituierten Reste ausgewählt sind aus C1 bis C10 Alkyl, C2 bis C10 Alkenyl, C2 bis C10 Alkinyl, Halogen, substituiertem oder nicht substituiertem Aryl, substituierten oder nicht substituierten heteroaromatischen Verbindungen, nicht aromatischen Heterocyclen, C1 bis C10 Alkyloxy, C1 bis C10 Alkylamin, C2 bis C10 Alkenylamino, C2 bis C10 Alkinylamino, COR⁶, CONR⁶ und COOR⁶_{;}
R⁶ ausgewählt ist aus H, unsubstituiertem oder substituiertem C1 bis C10 Alkyl, C2 bis C10 Alkenyl oder Alkinyl, Benzyl, Aryl, unsubstituierten oder substituierten heteroaromatischen Ringen mit einem oder mehreren Heteroatomen und nicht aromatischen Heterocyclen;
R⁷ und R⁸ zusammen eine verbrückende CH₂-CH₂-Einheit bilden, oder R⁷ und R⁸ beide Wasserstoff sind;
oder ein pharmazeutisch verträgliches Salz oder Prodrug davon,
zur Verwendung bei der Behandlung von malignem Melanom.

9. Verbindung gemäß Anspruch 8, wobei die Verbindung ausgewählt ist aus Verbindungen der folgenden Formel (II) 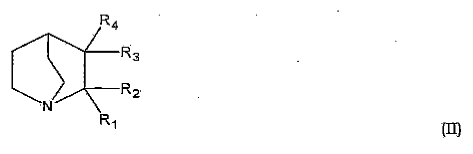 wobei
R₁ und R₂ unabhängig ausgewählt sind aus Wasserstoff, Hydroxymethyl oder einem Methylenrest, welcher verbunden ist mit dem Stickstoffatom eines Amin-substituierten Phenylrests, mit dem Stickstoffatom, welches in der Ringstruktur eines Purin-, 8-Azapurin- oder Senzimidazolrests enthalten ist, oder R₁ und R₂ zusammen einen doppelt gebundenen Methylenrest darstellen können, und
R₃ und R₄ unabhängig ausgewählt sind aus Wasserstoff, Hydroxyl und Benzoyloxy, oder R₃ und R₄ zusammen ein Sauerstoffatom, welches doppelt gebunden ist, darstellen können, mit der Maßgabe, dass wenn einer von R₃ und R₄ ein Benzyloxyrest ist, sowohl R₁ als auch R₂ Wasserstoff ist, oder einem pharmazeutisch verträglichen Salz oder Prodrug davon.

10. Verbindung gemäß Anspruch 9, wobei die Verbindung ausgewählt ist aus 2,2-Bis(hydroxymethyl)-1-azabicyclo[2.2.2]octan-3-on, 9-(Azabicyclo[2.2.2]octan-3-on)-6-chlor-9H-purin, 2-(Hydroxymethyl)chinuclidin-3,3-diol, 2-(Adenin-9-methylen)-3-chinuclidinon, 2-Methylen-3-chinuclidinon, 2-(2-Amino-3-chlor-5-trifluormethyl-1-methylanilin)-3-chinuclidinon, 2-(6-Trifluormethyl-4-chlorbenzimidazol-1-methylen)-3-chinuclidinon, 2-(6-Methoxypurin-9-methylen)-3-chinuclidinon, 2-(8-Azaadenin-9-methylen)-3-chinuclidinon, 1-Azabicymo[2.2.2]oct-3-ylbenzoat, 2-(5,6-Dimethylbenz-imidazol-1-methylen)-3-chinuclidinon, 2-(8-Azaadenin-7-methylen)-3-chinuclidinon, 2-(7-Methylen-1,3-dimethylharnsäure)-3-chinuclidinon oder 2-(2,6-Dichlor-9-methylen-purin)-3-chinuclidinon, oder einem pharmazeutisch verträglichen Salz davon.

11. Verbindung, welche Wildtyp p53 aus einer inaktiven Konformation davon, wobei die Konformation mit Pab 240 und nicht mit Pab 1620 reaktiv ist, in eine aktive Konformation überführen kann, welche Apoptose herbeiführen kann, wobei die Verbindung ausgewählt ist aus Verbindungen mit einer Struktur gemäß der Formel 1 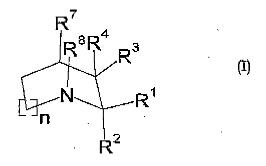 wobei
n 0, 1 oder 2 ist;
R¹ und R² gleich oder verschieden sind und ausgewählt sind aus -H, -OH₂-R⁵, -CH₂-O-R⁵, -CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ und -CH₂-O-CO-NHR⁵, oder R¹ und R² zusammen =CH₂ sind;
R³ und R⁴ gleich oder verschieden sind und ausgewählt sind aus -H, -OH, -SH, -NH₂, -NHR⁵ und -O-CO-C₆H₅, oder R³ und R⁴ zusammen =O, =S, =NH oder =NR⁵ sind;
R⁵ gleiche oder verschiedene Reste darstellt, ausgewählt aus H, substituiertem oder nicht substituiertem C1 bis C10 Alkyl, C2 bis C10 Alkenyl, C2 bis C10 Alkinyl, substituiertem oder nicht substituiertem C3 bis C12 Cycloalkyl, substituierten oder nicht substituierten Benzylresten, substituiertem oder nicht substituiertem Aryl oder mono-, bi-, tricyclischen(m) unsubstituierten(m) oder substituierten(m) heteroaromatischen(m) Ring(en) mit einem oder mehreren Heteroatomen und nicht aromatischen Heterocyclen, wobei
die Substituenten der substituierten Reste ausgewählt sind aus C1 bis C10 Alkyl, C2 bis C10 Alkenyl, C2 bis C10 Alkinyl, Halogen, substituiertem oder nicht substituiertem Aryl, substituierten oder nicht substituierten heteroaromatischen Verbindungen, nicht aromatischen Heterocyclen, C1 bis C10 Alkyloxy, C1 bis C10 Alkylamino, C2 bis C10 Alkenylamino, C2 bis C10 Alkinylamino, COR⁶, CONR⁶ und COOR⁶;
R⁶ ausgewählt ist aus H, unsubstituiertem oder substituiertem C1 bis C10 Alkyl, C2 bis C10 Alkenyl oder Alkinyl, Benzyl, Aryl, unsubstituierten oder substituierten heteroaromatischen Ringen mit einem oder mehreren Heteroatomen und nicht aromatischen Heterocyclen;
R⁷ und R⁸ zusammen eine verbrückende CH₂-CH₂-Einheit bilden, oder R⁷ und R⁸ beide Wasserstoff sind;
oder einem pharmazeutisch verträglichen Salz oder Prodrug davon,
mit der Maßgabe, dass wenn
n 1 ist;
R³ und R⁴ ausgewählt sind aus -O-CO-C₆H₅ und =O; und
R⁷ und R⁸ zusammen eine verbrückende CH₂-CH₂-Einheit bilden;
R¹ und R² nicht beide -H sind und nicht zusammen einen doppelt gebundenen Methylenrest =CH₂ oder einen einfach gebundenen Methylenrest bilden, welcher verbunden ist mit dem Stickstoffatom eines Amin-substituierten Phenylrests oder mit einem Stickstoffatom, welches in der Ringstruktur eines Purin-, 8-Azapurin- oder Benzimidazolrests enthalten ist;
zur Verwendung bei der Behandlung von malignem Melanom und/oder eines pathologischen Zustands, welcher unerwünschte Angiogenese einschließt.

12. Verbindung gemäß Anspruch 11, wobei die Verbindung ausgewählt ist aus Verbindungen der folgenden Formel (II) 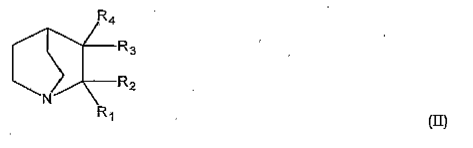 wobei
R₁ und R₂ unabhängig ausgewählt sind aus Wasserstoff, Hydroxymethyl oder einem Methylenrest, welcher verbunden ist mit dem Stickstoffatom eines Amin-substituierten Phenylrests, mit einem Stickstoffatom, welches in der Ringstruktur eines Purin-, 8-Azapurin- oder Benzimidazolrests enthalten ist, oder R₁ und R₂ zusammen einen doppelt gebundenen Methylenrest darstellen können, und
R₃ und R₄ unabhängig ausgewählt sind aus Wasserstoff, Hydroxyl und Benzoyloxy, oder R₃ und R₄ zusammen ein Sauerstoffatom, welches doppelt gebunden ist, darstellen können, mit der Maßgabe, dass wenn einer von R₃ und R₄ ein Benzyloxyrest ist, sowohl R₁ als auch R₂ Wasserstoff ist, oder einem pharmazeutisch verträglichen Salz oder Prodrug davon.

13. Verbindung gemäß Anspruch 12, wobei die Verbindung ausgewählt ist aus 2,2-Bis(hydroxymethyl)-1-azabicyclo[2.2.2]octan-3-on, 9-(Azabicyclo[2.2.2]octan-3-on)-6-chlor-9H-purin, 2-(Hydroxymethyl)chinuclidin-3,3-diol, oder einem pharmazeutisch verträglichen Salz davon.

14. Verbindung gemäß einem der Ansprüche 8 bis 13, zusammen mit einem pharmazeutisch verträglichen Träger, Verdünnungsmittel und/oder Exzipienten.

15. Verfahren zum Testen von Verbindungen auf ihre Fähigkeit, Wildtyp p53 von einer inaktiven Konformation in eine aktive Konformation zu überführen, umfassend die Schritte:
A. Bereitstellen von Zellen, welche nur wt p53 und kein mutiertes p53 enthalten, wobei in den Zellen die inaktive wt p53-Konformation vorhanden ist;
B. Aussetzen der Zellen in vitro einer zu testenden Substanz; und
C. Messen der zellulären inaktiven wt p53-Konformation.

16. Verfahren gemäß Anspruch 15, wobei statt des Schritts C ein alternativer Schritt C' angewendet wird, umfassend das Vergleichen des Effekts der getesteten Substanz auf die Zellen (die funktionelles p53 enthalten) in Schritt B mit dem Effekt auf die Zellen oder Gewebe mit keinem oder nicht funktionellen p53.

17. Verfahren gemäß Anspruch 15 oder 16, wobei Integrin αᵥβ₃ in den Zellen vorhanden ist.

18. Verfahren gemäß Anspruch 15 bis 17, wobei das Pab 240 für den Nachweis von wt p53 in seiner inaktiven Konformation verwendet wird.

19. Verfahren gemäß einem der Ansprüche 15 bis 18, wobei eine Verbindung mit einer Struktur der Formel I 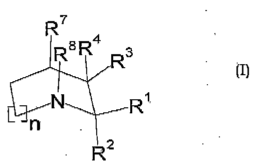 wobei
n 0, 1 oder 2 ist;
R¹ und R² gleich oder verschieden sind und ausgewählt sind aus -H, -CH₂-R⁵, -CH₂-O-R⁵, -CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ und -CH₂-O-CO-NHR⁵, oder R¹ und R² zusammen =CH₂ sind;
R³ und R⁴ gleich oder verschieden sind und ausgewählt sind aus -H, -OH, -SH, -NH₂, -NHR⁵ und -O-CO-C₆H₅, oder R³ und R⁴ zusammen =O, =S, =NH oder =NR⁵ sind;
R⁵ gleiche oder verschiedene Reste darstellt, ausgewählt aus H, substituiertem oder nicht substituiertem C1 bis C10 Alkyl, C2 bis C10 Alkenyl, C2 bis C10 Alkinyl, substituiertem oder nicht substituiertem C3 bis C12 Cycloalkyl-, substituierten oder nicht substituierten Benzylresten, substituiertem oder nicht substituiertem Aryl oder mono-, bi-, tricyclischen(m) unsubstituierten(m) oder substituierten(m) heteroaromatischen(in) Ring(en) mit einem oder mehreren Heteroatomen und nicht aromatischen Heterocyclen, wobei
die Substituenten der substituierten Reste ausgewählt sind aus C1 bis C10 Alkyl, C2 bis C10 Alkenyl, C2 bis C10 Alkinyl, Halogen, substituiertem oder nicht substituiertem Aryl, substituierten oder nicht substituierten heteroaromatischen Verbindungen, nicht aromatischen Heterocyclen, C1 bis C10 Alkyloxy, C1 bis C10 Alkylamin, C2 bis C10 Alkenylamino, C2 bis C10 Alkinylamino, COR⁶, CONR⁶ und COOR⁶;
R⁶ ausgewählt ist aus H, unsubstituiertem oder substituiertem C1 bis C10 Alkyl, C2 bis C10 Alkenyl oder Alkinyl, Benzyl, Aryl, unsubstituierten oder substituierten heteroaromatischen Ringen mit einem oder mehreren Heteroatomen und nicht aromatischen Heterocyclen;
R⁷ und R⁸ zusammen eine verbrückende CH₂-CH₂-Einheit bilden, oder R⁷ und R⁸ beide Wasserstoff sind;
oder ein pharmazeutisch verträgliches Salz oder Prodrug davon getestet wird.

20. Verfahren gemäß einem der Ansprüche 15 bis 19, wobei die Zellen in Schritt B in vivo einer zu testenden Substanz in einem Tier ausgesetzt werden und das Tier anschließen getötet wird.

## Revendications

1. Utilisation d'un composé capable de transférer p53 de type sauvage d'une conformation inactive de celui-ci, laquelle conformation est sensible à Pab 240 et non à Pab 1620, en une conformation active capable d'induire une apoptose, lequel composé est sélectionné parmi les composés ayant la structure selon la formule I 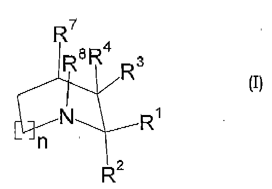 dans laquelle
n vaut 0, 1 ou 2 ;
R¹ et R² sont identiques ou différents et sont sélectionnés parmi -H, -CH₂-R⁵, -CH₂-O-R⁵,
-CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ et -CH₂-O-CO-NHR⁵, ou R¹ et R² sont conjointement =CH₂ ;
R³ et R⁴ sont identiques ou différents et sont sélectionnés parmi -H, -OH, -SH, -NH₂, -NHR⁵ et -O-CO-C₆H₅ ; ou R³ et R⁴ sont conjointement =O, =S, =NH ou =NR⁵ ;
R⁵ représente des groupes identiques ou différents sélectionnés parmi H, les groupes alkyle en C1 à C10 substitué ou non substitué, alcényle en C2 à C10, alcynyle en C2 à C10, cycloalkyle en C3 à C12 substitué ou non substitué, benzyle substitué ou non substitué, aryle substitué ou non substitué ou un/des noyau(x) hétéroaromatique(s) mono-, bi-, tricyclique(s) substitué(s) ou non substitué(s) avec un ou plusieurs hétéroatomes et hétérocycles non aromatiques dans lesquels
les substituants des groupes substitués sont sélectionnés parmi un alkyle en C1 à C10, un alcényle en C2 à C10, un alcynyle en C2 à C10, un halogène, un aryle substitué ou non substitué, des composés hétéroaromatiques substitués ou non substitués, des hétérocycles non aromatiques, un alkyloxy en C1 à C10, un alkylamino en C1 à C10, un alcénylamino en C2 à C10, un alcynylamino en C2 à C10, COR⁶, CONR⁶ et COOR⁶ ;
R⁶ est sélectionné parmi H, un alkyle en C1 à C10 substitué ou non substitué, un alcényle ou un alcynyle en C2 à C10, un benzyle, un aryle, des noyaux hétéroaromatiques substitués ou non substitués avec un ou plusieurs hétéroatomes et hétérocycles non aromatiques ;
R⁷ et R⁸ forment conjointement un groupe de pontage CH₂-CH₂ ; ou R⁷ et R⁸ sont tous deux de un hydrogène ;
ou un sel ou un promédicament pharmaceutiquement acceptable de celui-ci,
pour la préparation d'un médicament destiné à être utilisé dans le traitement d'un mélanome malin.

2. Utilisation selon la revendication 1, dans laquelle le composé est sélectionné parmi les composés ayant la formule suivante (II) 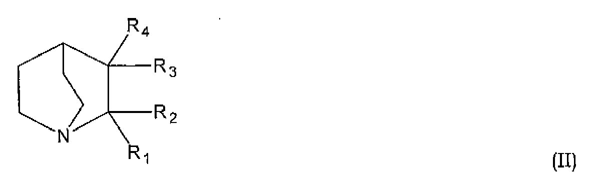 dans laquelle :
R₁ et R₂ sont indépendamment sélectionnés parmi un hydrogène, un hydroxyméthyle ou un groupe méthylène lié à l'atome d'azote d'un groupe phényle substitué par une amine, à un atome d'azote contenu dans la structure du noyau d'un résidu de purine, de 8-azapurine ou de benzimidazole, ou R₁ et R₂ peuvent représenter conjointement un groupe méthylène à double liaison, et ;
R₃ et R₄ sont indépendamment sélectionnés parmi un hydrogène, un hydroxyle et un benzoyloxy, ou R₃ et R₄ peuvent représenter conjointement un atome d'oxygène à double liaison, à condition que lorsque l'un des R₃ et R₄ est un groupe benzoyloxy, R₁ et R₂ sont tous deux un hydrogène, ou un sel ou un promédicament pharmaceutiquement acceptable de celui-ci.

3. Utilisation selon la revendication 2, dans laquelle le composé est sélectionné parmi la 2,2-bis(hydroxyméthyl)-1-azabicyclo[2.2.2]octan-3-one, la 9-(azabicyclo[2.2.2]octan-3-one)-6-chloro-9H-purine, le 2-(hydroxyméthyl)quinuclidine-3,3-diol, la 2-(adénine-9-méthylène)-3-quinuclidinone, la 2-méthylène-3-quinuclidinone, la 2-(2-amino-3-chloro-5-trifluorométhyl-1-méthylaniline)-3-quinuclidinone, la 2-(6-trifluorométhyl-4-chlorobenzimidazole-1-méthylène)-3-quinuclidinone, la 2-(6-méthoxypurine-9-méthylène)-3-quinuclidinone, la 2-(8-azaadénine-9-méthylène)-3-quinuclidinone, le benzoate de 1-azabicyclo[2.2.2]oct-3-yle, la 2-(5,6-diméthyl-benzimidazole-1-méthylène)-3-quinuclidinone, la 2-(8-azaadénine-7-méthylène)-3-quinuclidinone, la 2-(7-méthylène-1,3-acide diméthylurique)-3-quinuclidinone ou la 2-(2,6-dichloro-9-méthylènepurine)-3-quinuclidinone ou un sel pharmaceutiquement acceptable de celui-ci.

4. Utilisation d'un composé capable de transférer p53 de type sauvage d'une conformation inactive de celui-ci, laquelle conformation est sensible à Pab 240 et non à Pab 1620, en une conformation active capable d'induire une apoptose, lequel composé est sélectionné parmi les composés ayant la structure selon la formule I 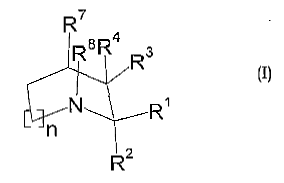 dans laquelle
n vaut 0, 1 ou 2 ;
R¹ et R² sont identiques ou différents et sont sélectionnés parmi -H, -CH₂-R⁵, -CH₂-O-R⁵,
-CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ et -CH₂-O-CO-NHR⁵ ; ou R¹ et R² sont conjointement =CH₂ ;
R³ et R⁴ sont identiques ou différents et sont sélectionnés parmi -H, -OH, -SH, -NH₂, -NHR⁵ et -O-CO-C₆H₅ ; ou R³ et R⁴ sont conjointement =O, =S, =NH ou =NR⁵ ;
R⁵ représente des groupes identiques ou différents sélectionnés parmi H, les groupes alkyle en C1 à C10 substitué ou non substitué, alcényle en C2 à C10, alcynyle en C2 à C10, cycloalkyle en C3 à C12 substitué ou non substitué, benzyle substitué ou non substitué, aryle substitué ou non substitué ou un/des noyau(x) hétéroaromatique(s) mono-, bi-, tricyclique(s) substitué(s) ou non substitué(s) avec un ou plusieurs hétéroatomes et hétérocycles non aromatiques dans lesquels
les substituants des groupes substitués sont sélectionnés parmi un alkyle en C1 à C10, un alcényle en C2 à C10, un alcynyle en C2 à C10, un halogène, un aryle substitué ou non substitué, des composés hétéroaromatiques substitués ou non substitués, des hétérocycles non aromatiques, un alkyloxy en C1 à C10, un alkylamino en C1 à C10, un alcénylamino en C2 à C10, un alcynylamino en C2 à C10, COR⁶, CONR⁶ et COOR⁶ ;
R⁶ est sélectionné parmi H, un alkyle en C1 à C10 substitué ou non substitué, un alcényle ou un alcynyle en C2 à C10, un benzyle, un aryle, des noyaux hétéroaromatiques substitués ou non substitués avec un
ou plusieurs hétéroatomes et hétérocycles non aromatiques ;
R⁷ et R⁸ forment conjointement un groupe de pontage CH₂-CH₂ ; ou R⁷ et R⁸ sont tous deux un hydrogène ;
ou un sel ou un promédicament pharmaceutiquement acceptable de celui-ci,
à condition que lorsque
n vaut 1 ;
R³ et R⁴ sont sélectionnés parmi O-CO-C₆H₅ et =O ; et
R⁷ et R⁸ forment conjointement un groupe de pontage CH₂-CH₂ ;
R¹ et R² ne sont pas tous les deux -H et ne forment pas conjointement un groupe méthylène à double liaison =CH₂ ou un groupe méthylène à simple liaison lié à l'atome d'azote d'un groupe phényle substitué par une amine, ou à un atome d'azote contenu dans la structure du noyau d'un résidu de purine, de 8-azapurine ou de benzimidazole ;
pour la préparation d'un médicament destiné à être utilisé dans le traitement d'un mélanome malin et/ou d'une condition pathologique impliquant une angiogenèse indésirable.

5. Utilisation selon la revendication 4, dans laquelle le composé est sélectionné parmi les composés de formule suivante (II) 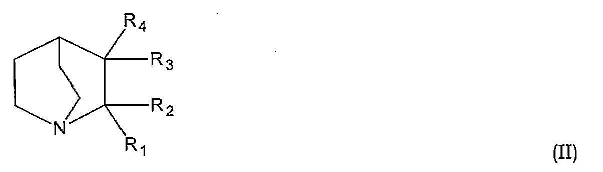 dans laquelle :
R₁ et R₂ sont indépendamment sélectionnés parmi un hydrogène, un hydroxyméthyle ou un groupe méthylène lié à l'atome d'azote d'un groupe phényle substitué par une amine, à un atome d'azote contenu dans la structure du noyau d'un résidu de purine, de 8-azapurine ou de benzimidazole, ou R₁ et R₂ peuvent représenter conjointement un groupe méthylène à double liaison, et ;
R₃ et R₄ sont indépendamment sélectionnés parmi un hydrogène, un hydroxyle, et un benzoyloxy, ou R₃ et R₄ peuvent représenter conjointement un atome d'oxygène à double liaison, à condition que lorsque l'un de R₃ et R₄ est un groupe benzoyloxy, R₁ et R₂ sont tous deux un hydrogène, ou un sel ou un promédicament pharmaceutiquement acceptable de celui-ci.

6. Utilisation selon la revendication 5, dans laquelle le composé est sélectionné parmi la 2,2-bis(hydroxyméthyl)-1-azabicyclo[2.2.2]octan-3-one, la 9-(azabicyclo[2.2.2]octan-3-one)-6-chloro-9H-purine, le 2-(hydroxyméthyl)quinuclidine-3,3-diol ou un sel pharmaceutiquement acceptable de celui-ci.

7. Utilisation selon l'une quelconque des revendications 1 à 6 conjointement avec un support, diluant et/ou excipient pharmaceutiquement acceptable.

8. Composé capable de transférer p53 de type sauvage d'une conformation inactive de celui-ci, laquelle conformation est sensible à Pab 240 et non à Pab 1620, en une conformation active capable d'induire une apoptose, lequel composé est sélectionné parmi les composés ayant la structure selon la formule I 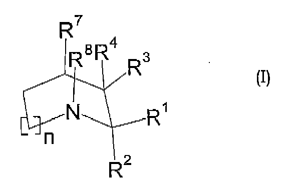 dans laquelle
n vaut 0, 1 ou 2 ;
R¹ et R² sont identiques ou différents et sont sélectionnés parmi -H, -CH₂-R⁵, -CH₂-O-R⁵,
-CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ et -CH₂-O-CO-NHR⁵ ; ou R¹ et R² sont conjointement =CH₂ ;
R³ et R⁴ sont identiques ou différents et sont sélectionnés parmi -H, -OH, -SH, -NH₂, -NHR⁵ et -O-CO-C₆H₅ ; ou R³ et R⁴ sont conjointement =O, =S, =NH ou =NR⁵ ;
R⁵ représente des groupes identiques ou différents sélectionnés parmi H, les groupes alkyle en C1 à C10 substitué ou non substitué, alcényle en C2 à C10, alcynyle en C2 à C10, cycloalkyle en C3 à C12 substitué ou non substitué, benzyle substitué ou non substitué, aryle substitué ou non substitué ou un/des noyau(x) hétéroaromatique(s) mono-, bi-, tricyclique(s) substitué(s) ou non substitué(s) avec un ou plusieurs hétéroatomes et hétérocycles non aromatiques dans lesquels
les substituants des groupes substitués sont sélectionnés parmi un alkyle en C1 à C10, un alcényle en C2 à C10, un alcynyle en C2 à C10, un halogène, un aryle substitué ou non substitué, des composés hétéroaromatiques substitués ou non substitués, des hétérocycles non aromatiques, un alkyloxy en C1 à C10, un alkylamino en C1 à C10, un alcénylamino en C2 à C10, un alcynylamino en C2 à C10, COR⁶, CONR⁶ et COOR⁶ ;
R⁶ est sélectionné parmi H, un alkyle en C1 à C10 substitué ou non substitué, un alcényle ou un alcynyle en C2 à C10, un benzyle, un aryle, des noyaux hétéroaromatiques substitués ou non substitués avec un
ou plusieurs hétéroatomes et hétérocycles non aromatiques :
R⁷ et R⁸ forment conjointement un groupe de pontage CH₂-CH₂ ; ou R⁷ et R⁸ sont tous deux un hydrogène ;
ou un sel ou un promédicament pharmaceutiquement acceptable de celui-ci,
pour une utilisation dans le traitement d'un mélanome malin.

9. Composé selon la revendication 8, lequel composé est sélectionné parmi les composés de formule suivante (II) 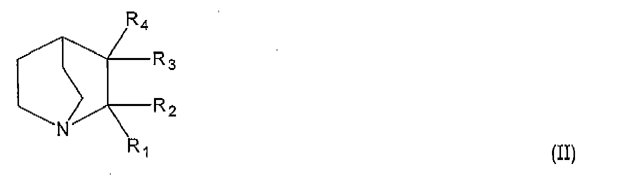 dans laquelle :
R₁ et R₂ sont indépendamment sélectionnés parmi un hydrogène, un hydroxyméthyle ou un groupe méthylène lié à l'atome d'azote d'un groupe phényle substitué par une amine, à un atome d'azote contenu dans la structure du noyau d'un résidu de purine, de 8-azapurine ou de benzimidazole, ou R₁ et R₂ peuvent représenter conjointement un groupe méthylène à double liaison, et ;
R₃ et R₄ sont indépendamment sélectionnés parmi un hydrogène, un hydroxyle et un benzoyloxy, ou R₃ et R₄ peuvent représenter conjointement un atome d'oxygène à double liaison, à condition que lorsque l'un des R₃ et R₄ est un groupe benzoyloxy, R₁ et R₂ sont tous deux un hydrogène, ou un sel ou un promédicament pharmaceutiquement acceptable de celui-ci.

10. Composé selon la revendication 9, dans lequel le composé est sélectionné parmi la 2,2-bis(hydroxyméthyl)-1-azabicyclo[2.2.2]octan-3-one, la 9-(azabicyclo[2.2.2]octan-3-one)-6-chloro-9H-purine, le 2-(hydroxyméthyl)quinuclidine-3,3-diol, la 2-(adénine-9-méthylène)-3-quinuclidinone, la 2-méthylène-3-quinuclidinone, la 2-(2-amino-3-chloro-5-trifluorométhyl-1-méthylaniline)-3-quinuclidinone, la 2-(6-trifluorométhyl-4-chlorobenzimidazole-1-méthylène)-3-quinuclidinone, la 2-(6-méthoxypurine-9-méthylène)-3-quinuclidinone, la 2-(8-azaadénine-9-méthylène)-3-quinuclidinone, le benzoate de 1-azabicyclo[2.2.2]oct-3-yle, la 2-(5,6-diméthylbenzimidazole-1-méthylène)-3-quinuclidinone, la 2-(8-azaadénine-7-méthylène)-3-quinuclidinone, la 2-(7-méthylène-1,3-acide diméthylurique)-3-quinuclidinone ou la 2-(2,6-dichloro-9-méthylènepurine)-3-quinuclidinone ou un sel pharmaceutiquement acceptable de celui-ci.

11. Composé capable de transférer p53 de type sauvage d'une conformation inactive de celui-ci, laquelle conformation est sensible à Pab 240 et non à Pab 1620, en une conformation active capable d'induire une apoptose, lequel composé est sélectionné parmi les composés ayant la structure selon la formule I 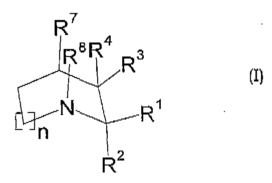 dans laquelle
n vaut 0, 1 ou 2 ;
R¹ et R² sont identiques ou différents et sont sélectionnés parmi -H, -CH₂-_{R}⁵, -CH₂-O-R⁵,
-CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ et -CH₂-O-CO-NHR⁵ ; ou R¹ et R² sont conjointement =CH₂ ;
R³ et R⁴ sont identiques ou différents et sont sélectionnés parmi -H, -OH, -SH, -NH₂, -NHR⁵ et -O-CO-C₆H₅ ; ou R³ et R⁴ sont conjointement =O, =S, =NH ou =NR⁵ ;
R⁵ représente des groupes identiques ou différents sélectionnés parmi H, les groupes alkyle en C1 à C10 substitué ou non substitué, alcényle en C2 à C10, alcynyle en C2 à C10, cycloalkyle en C3 à C12 substitué ou non substitué, benzyle substitué ou non substitué, aryle substitué ou non substitué ou un/des noyau(x) hétéroaromatique(s) mono-, bi-, tricyclique(s) substitué(s) ou non substitué(s) avec un ou plusieurs hétéroatomes et hétérocycles non aromatiques dans lesquels
les substituants des groupes substitués sont sélectionnés parmi un alkyle en C1 à C10, un alcényle en C2 à C10, un alcynyle en C2 à C10, un halogène, un aryle substitué ou non substitué, des composés hétéroaromatiques substitués ou non substitués, des hétérocycles non aromatiques, un alkyloxy en C1 à C10, un alkylamino en C1 à C10, un alcénylamino en C2 à C10, un alcynylamino en C2 à C10, COR⁶, CONR⁶ et COOR⁶ ;
R⁶ est sélectionné parmi H, un alkyle en C1 à C10 substitué ou non substitué, un alcényle ou un alcynyle en C2 à C10, un benzyle, un aryle, des noyaux hétéroaromatiques substitués ou non substitués avec un ou plusieurs hétéroatomes et hétérocycles non aromatiques ;
R⁷ et R⁸ forment conjointement un groupe de pontage CH₂-CH₂ ; ou R⁷ et R⁸ sont tous deux un hydrogène ;
ou un sel ou un promédicament pharmaceutiquement acceptable de celui-ci,
à condition que lorsque
n vaut 1 ;
R³ et R⁴ sont sélectionnés parmi O-CO-C₆H₅ et =O ; et
R⁷ et R⁸ forment conjointement un groupe de pontage CH₂-CH₂ ;
R¹ et R² ne sont pas tous les deux -H et ne forment pas conjointement un groupe méthylène à double liaison =CH₂ ou un groupe méthylène à simple liaison lié à l'atome d'azote d'un groupe phényle substitué par une amine, ou à un atome d'azote contenu dans la structure du noyau d'un résidu de purine, de 8-azapurine ou de benzimidazole ;
pour une utilisation dans le traitement d'un mélanome malin et/ou d'une condition pathologique impliquant une angiogenèse indésirable.

12. Composé selon la revendication 11, lequel composé est sélectionné parmi les composés de formule suivante (II) 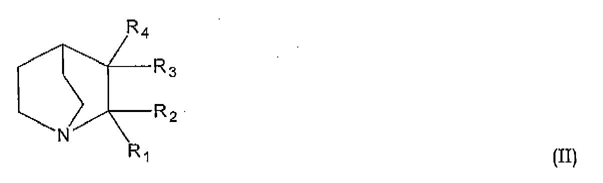 dans laquelle :
R₁ et R₂ sont indépendamment sélectionnés parmi un hydrogène, un hydroxyméthyle, ou un groupe méthylène lié à l'atome d'azote d'un groupe phényle substitué par une amine, à un atome d'azote contenu dans la structure du noyau d'un résidu de purine, de 8-azapurine ou de benzimidazole, ou R₁ et R₂ peuvent représenter conjointement un groupe méthylène à double liaison, et ;
R₃ et R₄ sont indépendamment sélectionnés parmi un hydrogène, un hydroxyle et un benzoyloxy, ou R₃ et R₄ peuvent représenter conjointement un atome d'oxygène à double liaison, à condition que lorsque l'un des R₃ et R₄ est un groupe benzoyloxy, R₁ et R₂ sont tous deux un hydrogène, ou un sel ou un promédicament pharmaceutiquement acceptable de celui-ci.

13. Composé selon la revendication 12, lequel composé est sélectionné parmi la 2,2-bis(hydroxyméthyl)-1-azabicyclo[2.2.2]octan-3-one, la 9-(azabicyclo[2.2.2]octan-3-one)-6-chloro-9H-purine, le 2-(hydroxyméthyl)quinuclidine-3,3-diol ou un sel pharmaceutiquement acceptable de celui-ci.

14. Composé selon l'une quelconque des revendications 8 à 13 conjointement avec un support, diluant et/ou excipient pharmaceutiquement acceptable.

15. Procédé de test de composés quant à la capacité de transférer p53 de type sauvage d'une conformation inactive en une conformation active comprenant les étapes de :
A. Fourniture de cellules portant uniquement p53 wt et non p53 mutant, dans lesquelles cellules la conformation inactive de p53 wt est présente ;
B. Exposition des cellules in vitro à une substance devant être testée ; et
C. Mesure de la conformation inactive cellulaire de p53 wt.

16. Procédé selon la revendication 15, dans lequel à la place de l'étape C, une étape alternative C' est utilisée, comprenant la comparaison de l'effet de la substance testée sur les cellules (portant p53 fonctionnel) dans l'étape B par rapport à l'effet sur les cellules ou les tissus sans p53 ou avec p53 non fonctionnel.

17. Procédé selon la revendication 15 ou 16, dans lequel de l'intégrine αᵥβ₃ est présente dans les cellules.

18. Procédé selon les revendications 15 à 17, dans lequel le Pab 240 est utilisé pour détecter p53 wt dans sa conformation inactive.

19. Procédé selon l'une quelconque des revendications 15 à 18, dans lequel un composé ayant la structure selon la formule I 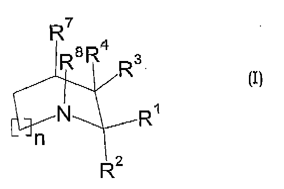 dans laquelle
n vaut 0, 1 ou 2 ;
R¹ et R² sont identiques ou différents et sont sélectionnés parmi -H, -CH₂-R⁵, -CH₂-O-R⁵,
-CH₂-S-R⁵, -CH₂-NH-R⁵, -CO-O-R⁵, -CO-NH-R⁵, -CH₂-NH-CO-R⁵, -CH₂-O-CO-R⁵, -CH₂-NH-CO-NHR⁵, -CH₂-NH-CO-OR⁵, -CH₂-NH-CS-NHR⁵ et -CH₂-O-CO-NHR⁵ ; ou R¹ et R² sont conjointement =CH₂ ;
R³ et R⁴ sont identiques ou différents et sont sélectionnés parmi -H, -OH, -SH, -NH₂, -NHR⁵ et -O-CO-C₆H₅ ; et R³ et R⁴ sont conjointement =O, =S, =NH ou =NR⁵ ;
R⁵ représente des groupes identiques ou différents sélectionnés parmi H, les groupes alkyle en C1 à C10 substitué ou non substitué, alcényle en C2 à C10, alcynyle en C2 à C10, cycloalkyle en C3 à C12 substitué ou non substitué, benzyle substitué ou non substitué, aryle substitué ou non substitué ou un/des noyau(x) hétéroaromatique(s) mono-, bi-, tricyclique(s) substitué(s) ou non substitué(s) avec un ou plusieurs hétéroatomes et hétérocycles non aromatiques dans lesquels
les substituants des groupes substitués sont sélectionnés parmi un alkyle en C1 à C10, un alcényle en C2 à C10, un alcynyle en C2 à C10, un halogène, un aryle substitué ou non substitué, des composés hétéroaromatiques substitués ou non substitués, des hétérocycles non aromatiques, un alkyloxy en C1 à C10, un alkylamino en C1 à C10, un alcénylamino en C2 à C0, un alcynylamino en C2 à C10, COR⁶, CONR⁶ et COOR⁶ ;
R⁶ est sélectionné parmi H, un alkyle en C1 à C10 substitué ou non substitué, un alcényle ou un alcynyle en C2 à C10, un benzyle, un aryle, des noyaux hétéroaromatiques substitués ou non substitués avec un ou plusieurs hétéroatomes et hétérocycles non aromatiques ;
R⁷ et R⁸ forment conjointement un groupe de pontage CH₂-CH₂ ; ou R⁷ et R⁸ sont tous deux un hydrogène ;
ou un sel ou un promédicament pharmaceutiquement acceptable de celui-ci est testé.

20. Procédé selon l'une quelconque des revendications 15 à 19, dans lequel les cellules dans l'étape B sont exposées *in vivo* chez un animal à la substance devant être testée, et l'animal est ensuite sacrifié.
